# EUROPÄISCHE PATENTSCHRIFT

(11) **EP 3 284 714 B1**
(45) Veröffentlichungstag und Bekanntmachung des Hinweises auf die Patenterteilung: **15.05.2019**
(21) Anmeldenummer: 17187125.4
(22) Anmeldetag: 21.08.2017
(51) Int. Cl.: B81C 1/00

(54) **VERFAHREN ZUM HERSTELLEN EINES HOHLRAUMS MIT PORÖSER STRUKTUR**
METHOD FOR PRODUCING A CAVITY WITH POROSE STRUCTURE
PROCÉDÉ DE FABRICATION D'UNE CAVITÉ À STRUCTURE POREUSE

(30) Priorität: 19.08.2016 DE 102016215617
(43) Veröffentlichungstag der Anmeldung: 21.02.2018
(73) Patentinhaber: Fraunhofer-Gesellschaft zur Förderung der angewandten Forschung e.V., 80686 München (DE)
(72) Erfinder: LISEC, Thomas, 25524 Itzehoe (DE); LOFINK, Fabian, 25524 Itzehoe (DE)
(74) Vertreter: Schenk, Markus

(56) Entgegenhaltungen:
- DE-A1- 10 118 568
- DE-A1-102007 029 445
- DE-A1-102011 010 899
- US-A1- 2007 134 939

## Beschreibung

Die Erfindung betrifft ein Verfahren zum Herstellen eines Hohlraums mit poröser Struktur gemäß den Merkmalen von Anspruch 1, sowie eine Vorrichtung mit poröser Struktur und einem Hohlraum gemäß den Merkmalen von Anspruch 14. Die Herstellung poröser Keramiken oder Metalle an sich ist industriell etabliert. Es sind viele Verfahren bekannt, vom Sintern von Pulvern bis zur Pyrolyse von Keramik-Polymer-Schäumen. Allen Verfahren ist gemein, dass Temperaturen von zumeist deutlich über 400°C benötigt werden. Ferner entstehen Rohlinge des porösen Materials, die durch weitere Bearbeitungsschritte zuerst in eine für eine bestimmte Anwendung erforderliche Form gebracht und dann in einen benötigten Hohlraum integriert werden müssen.

Ein vorteilhaftes Verfahren zum erzeugen poröser Strukturen ist in der DE 10 2011 010 899 A1 beschrieben. Hier werden lose Partikel in oder auf ein Trägerelement ein- bzw. aufgebracht. Eine Beschichtung verfestigt die losen Partikel zu einer zusammenhängenden porösen Struktur. Eine spezielle Ausführungsform der DE 10 2011 010 899 A1 beschreibt das Herstellen eines Hohlraums mittels zweier Substrathälften unter Entfernung der gesamten porösen Struktur. Eine untere Substrathälfte wird mit losen Partikeln gefüllt, die vollständig zu einer Verbundstruktur verfestigt werden. Ein weiteres Material, das später eine Membran bildet, kann auf die Verbundstruktur aufgetragen werden. Eine obere Substrathälfte wird auf die untere Substrathälfte aufgesetzt und die Verbundstruktur wird anschließend vollständig entfernt, so dass nur noch die aus dem anderen Material bestehende Membran übrig bleibt. Alternativ kann die aus dem anderen Material bestehende Membran weggelassen werden, so dass nach dem vollständigen Entfernen der Verbundstruktur ein vollständig leerer Hohlraum zwischen den beiden Substrathälften gebildet wird.

Die Herstellung solcher Hohlräume benötigt viele einzelne Prozessschritte und ist deshalb aufwendig zu realisieren. Ein solches Verfahren ist somit nur unter erhöhtem Zeit- und Kostenaufwand realisierbar.

Demnach ist es wünschenswert, ein Verfahren bereitzustellen, mittels dem ein Hohlraum mit einer porösen Struktur einfach, d.h. schnell und kostengünstig herstellbar ist.

Diese Aufgabe wird erfindungsgemäß durch ein Verfahren mit den Merkmalen von Anspruch 1 gelöst.

Gemäß dem erfindungsgemäßen Verfahren wird zunächst ein Substrat, das eine Aussparung aufweist, bereitgestellt. Eine Vielzahl loser Partikel wird in die Aussparung eingebracht. Unter Verwendung eines Beschichtungsprozesses wird ein erster Anteil der Partikel beschichtet, und zwar so, dass der erste Anteil zu einer verfestigten porösen Struktur verbunden wird. Der Beschichtungsprozess weist hierfür eine Eindringtiefe auf, die von einer Öffnung der Aussparung ausgehend entlang einer Tiefenrichtung in die Aussparung hinein verläuft. In anderen Worten dringt die Beschichtung von oben in Richtung der Aussparung hinein in die Partikel ein. Dabei wird die Eindringtiefe des Beschichtungsprozesses in die Aussparung so eingestellt, dass ein zweiter Anteil der Partikel nicht mittels der Beschichtung verbunden wird. Das heißt, lediglich ein Teil bzw. ein Abschnitt der in der Aussparung befindlichen Partikel wird mittels der Beschichtung verfestigt, vorzugsweise der oben aufliegende Teil der Partikel. Der zu der porösen Struktur verfestigte erste Anteil der Partikel ist somit zwischen dem zweiten Anteil der nicht verfestigten losen Partikel und einer Umgebung der Aussparung angeordnet. In anderen Worten wird der nicht verfestigte zweite Anteil der Partikel, d.h. die nicht verfestigten losen Partikel, in einem Hohlraum, der sich zwischen der Aussparung und dem zu der porösen Struktur verfestigten ersten Anteil der Partikel ausbildet, eingeschlossen. Der Hohlraum wird also zwischen der in dem Substrat vorgesehenen Aussparung und der porösen Struktur gebildet. Die poröse Struktur stellt sozusagen einen Deckel der ansonsten offenen Aussparung bereit. Der Hohlraum ist zu diesem Zeitpunkt vollständig mit dem nicht verfestigten zweiten Anteil der Partikel gefüllt. Erfindungsgemäß wird dieser zweite Anteil der Partikel, d.h. die unter der porösen Struktur befindlichen nicht verfestigten losen Partikel, zumindest teilweise aus der Aussparung entfernt. Unter einem teilweise Entfernen des zweiten Anteils der Partikel ist zu verstehen, dass ein gewisser Anteil der ursprünglichen Anzahl des zweiten Anteils der Partikel aus der Aussparung entfernt wird. Wie eingangs erwähnt, ist der zweite Anteil der Partikel in dem zwischen der Aussparung und der porösen Struktur ausgebildeten Hohlraum eingeschlossen. Die nicht verfestigten losen Partikel, d.h. der zweite Anteil der Partikel, sind hierbei dicht gepackt. Oder anders ausgedrückt, die losen Partikel verteilen sich über den gesamten Hohlraum und füllen das gesamte Volumen des Hohlraums aus. Zumindest ein Teil dieser losen Partikel wird nun entfernt, so dass zwischen den einzelnen Partikeln größere Freiräume entstehen im Vergleich zur ursprünglichen Füllmenge. Dementsprechend füllen die verbleibenden losen Partikel weniger Volumen in dem Hohlraum aus. In dem Hohlraum befinden sich also nach dem Entfernen eines Teils der nicht verfestigten losen Partikel (zweiter Anteil der Partikel) weniger Partikel als ursprünglich in die Aussparung eingebracht wurden. Gleichzeitig bleibt aber der erste Anteil der Partikel, d.h. die zu der porösen Struktur verfestigten Partikel, bestehen. Es werden also selektiv nur die im Hohlraum eingeschlossenen nicht verfestigten losen Partikel entfernt, während hingegen die poröse Struktur erhalten bleibt. Die Beschichtung, die zur Verfestigung der losen Partikel und somit zu der Ausbildung der porösen Struktur führt, kann außerdem die poröse Struktur mit dem Substrat bzw. mit der Aussparung des Substrats koppeln, sodass die poröse Struktur mit dem Substrat verbunden bzw. an dem Substrat fixiert ist. Durch die poröse Struktur hindurch kann ein das Substrat umgebendes Medium, wie z.B. Luft oder Wasser, in die Aussparung hinein diffundieren.

Es ist vorstellbar, dass mindestens 10% oder mindestens 25% des zweiten Anteils der Partikel aus der Aussparung entfernt werden. Somit kann eine Auflockerung der zuvor dicht gepackten losen Partikel erfolgen. Es wird demnach ein Hohlraum geschaffen, der zumindest teilweise mit losen Partikeln gefüllt ist, wobei der Hohlraum eine poröse Struktur aufweist, durch die z.B. Flüssigkeiten oder Gase diffundieren können, um so eine fluidische Kommunikation des Hohlraums mit der Umgebung zu ermöglichen.

Gemäß einer weiteren denkbaren Ausführungsform können mehr als 50% des zweiten Anteils von Partikeln aus der Aussparung entfernt werden. Das heißt, der Hohlraum ist nur noch zu weniger als der Hälfte mit losen Partikeln gefüllt. Die in dem Hohlraum verbleibenden losen Partikel haben somit viel Freiraum, um sich in dem Hohlraum zu bewegen.

Es ist denkbar, dass der zweite Anteil der Partikel im Wesentlichen vollständig entfernt wird. Das heißt, die in dem Hohlraum eingeschlossenen nicht verfestigten losen Partikel werden vollständig aus dem Hohlraum entfernt. Somit kann ein Hohlraum bereitgestellt werden, der im Wesentlichen frei von Partikeln ist.

Der zweite Anteil der Partikel, d.h. die nicht verfestigten losen Partikel, kann mittels eines Ätzverfahrens entfernt werden. Hierfür können beispielsweise gängige nass- oder trockenchemische Ätzprozesse verwendet werden. Das jeweilige Ätzmittel kann beispielsweise durch die poröse Struktur hindurch in die Aussparung hinein diffundieren, um die darunterliegenden losen Partikel wegzuätzen. Es ist aber auch denkbar, dass das Ätzmittel durch eine in dem Substrat vorgesehene Öffnung in die Aussparung hinein eingebracht wird.

Es ist vorstellbar, dass der erste Anteil der Partikel ein anderes Material aufweist als der zweite Anteil der Partikel. Das heißt, die verfestigte poröse Struktur weist Partikel auf, die aus einem anderen Material (z.B. Material C) bestehen als die in dem Hohlraum eingeschlossenen unverfestigten losen Partikel (z.B. Material A und/oder Material B). Dies ist insofern von Vorteil, als dass ein Ätzmittel, das geeignet ist, um die unverfestigten losen Partikel zu entfernen, die poröse Struktur nicht angreift.

Gemäß einer Ausführungsform können zuerst lose Partikel, die ein erstes Material aufweisen, und anschließend lose Partikel, die ein zweites Material aufweisen, in die Aussparung eingebracht werden, wobei die das zweite Material aufweisenden Partikel anschließend beschichtet werden. Das heißt, es können beispielsweise zuerst lose Partikel aus einem Material A in die Aussparung eingebracht werden. Anschließend werden lose Partikel aus einem Material B auf die losen Partikel aus dem Material A aufgebracht. Die losen Partikel aus dem Material B liegen also auf den losen Partikeln aus dem Material A auf. Die Beschichtung kann dergestalt sein, dass sie lediglich die oben aufliegenden, aus dem Material B bestehenden, Partikel zu einer porösen Struktur verfestigt. Die darunterliegenden losen Partikel aus dem Material A hingegen werden nicht von der Beschichtung verfestigt, da die Beschichtung entweder nicht bis zu ihnen vordringt, oder weil das Material A nicht mit der Beschichtung reagiert um eine poröse Struktur auszubilden. Somit kann also eine selektive Verfestigung loser Partikel bereitgestellt werden. Außerdem kann ein Ätzmittel in den Hohlraum eingebracht werden, das nur die unverfestigten losen Partikel aus dem Material A auflöst, während die poröse Struktur aus den verfestigten Partikeln des Materials B nicht von dem Ätzmittel angegriffen werden.

Es ist ferner vorstellbar, dass eine Beschichtung, insbesondere eine Passivierungsschicht, auf die poröse Struktur aufgebracht wird, sodass sich die Beschichtung zumindest abschnittsweise über das Substrat und/oder zumindest abschnittsweise über die poröse Struktur erstreckt. Beispielsweise kann eine Passivierungsschicht aufgebracht werden, die das Substrat und einen in Substratnähe angeordneten Abschnitt der porösen Struktur bedeckt. Die Mitte bzw. ein innenliegender Teil der porösen Struktur kann ausgespart werden, so dass weiterhin Gase, Flüssigkeiten, etc. durch den ausgesparten Teil der porösen Struktur hindurch diffundieren können. In anderen Worten wird in der Passivierungsschicht eine Öffnung freigelassen, durch die die poröse Struktur in Kontakt mit der Umgebung bleibt.

Die Beschichtung kann aber auch derart auf die poröse Struktur aufgebracht werden, dass sie die poröse Struktur vollständig bedeckt und die poröse Struktur fluiddicht versiegelt. Eine fluiddichte Versiegelung kann vorteilhaft sein, um den Hohlraum bzw. die Aussparung vor dem Eindringen von Feuchtigkeit und/oder Schmutz zu bewahren.

Es ist denkbar, dass auf mindestens einer Innenseite der Aussparung zumindest abschnittsweise eine Schicht aufgebracht wird, die Adhäsionskräfte und/oder Reibungskräfte zwischen der Aussparung und einem nach dem zumindest teilweisen Entfernen des zweiten Anteils der Partikel in der Aussparung verbleibenden Partikels und/oder Objekts verringert, und/oder eine Schicht, die von einem nach dem zumindest teilweisen Entfernen des zweiten Anteils der Partikel in der Aussparung verbleibenden Partikels und/oder Objekts ausgehende Strahlung verringert. Die in dem Substrat vorgesehene Aussparung weist, wie üblich, eine Unterseite bzw. einen Boden und umlaufende Seitenwände auf, die auch insgesamt als Innenwände bezeichnet werden können. Die Beschichtung kann beispielsweise lediglich auf der Unterseite oder auf einer der Seitenwände aufgebracht werden. Die Beschichtung kann aber auch auf allen Innenseiten der Aussparung aufgebracht werden. Die Beschichtung kann beispielsweise dazu dienen, um ein Anhaften von in dem Hohlraum befindlichen Partikeln und/oder Objekten zu reduzieren bzw. vermeiden, d.h. die Beschichtung kann beispielsweise eine Antihaftbeschichtung sein. Die Beschichtung kann aber auch eine strahlungsreduzierende bzw. strahlungsabschirmende Funktion aufweisen. Die Beschichtung kann beispielsweise eine Metallbeschichtung sein.

Es ist vorstellbar, dass die Vielzahl loser Partikel Partikel aufweist, die ein magnetisches Material aufweisen. Das heißt, unter der Vielzahl loser Partikel können sich ein oder mehrere Partikel befinden, die ein magnetisches Material aufweisen oder aus einem magnetischen Material bestehen. So können beispielsweise neben der Vielzahl unverfestigter loser Partikel in dem Hohlraum zusätzlich ein oder mehrere magnetische Partikel als unverfestigte lose Partikel in dem Hohlraum vorliegen. Bei dem Prozessschritt des Entfernens der losen Partikel können beispielsweise lediglich die nicht magnetischen losen Partikel entfernt werden, so dass nur die magnetischen losen Partikel in dem Hohlraum übrig bleiben. So wird auf einfache Weise ein Hohlraum mit darin eingeschlossenen magnetischen Partikeln bereitgestellt, wobei der Hohlraum noch dazu von einer porösen Struktur abgedeckt ist.

Es ist denkbar, dass zur Herstellung der porösen Struktur der Beschichtungsprozess, mittels dem der erste Anteil der Partikel beschichtet wird, ein ALD-Prozess (Atomic Layer Deposition = Atomlagenabscheidung) ist. Dieser Prozess ist selbstbegrenzend, so dass eine sehr gute Prozessführung hinsichtlich der Ausbildung der Schichtdicke des Beschichtungsprozesses möglich ist. Das heißt, die Dicke der porösen Struktur kann mittels eines ALD-Prozesses sehr genau bestimmt werden.

Gemäß einer Ausführungsform des erfindungsgemäßen Verfahrens kann vor dem Beschichten des ersten Anteils der Partikel mindestens ein Objekt zusätzlich zu der Vielzahl von losen Partikeln in die Aussparung eingebracht werden. Beispielsweise kann die Vielzahl loser Partikel gleichzeitig zusammen mit dem mindestens einen Objekt, d.h. in Form eines Gemisches, in die Aussparung eingefüllt werden. Denkbar wäre aber auch, dass zuerst eine Schicht loser Partikel in die Aussparung eingebracht wird, anschließend das mindestens eine Objekt, und wieder anschließend eine weitere Schicht loser Partikel, so dass das mindestens eine Objekt von zwei oder mehr Schichten loser Partikel umschlossen ist. Das mindestens eine Objekt und die losen Partikel werden sozusagen entweder gleichzeitig oder nacheinander in die Aussparung eingebracht.

Es ist vorstellbar, dass das mindestens eine Objekt ein Partikel ist, das ein von der Vielzahl von losen Partikeln unterschiedliches Material aufweist, oder wobei das mindestens eine Objekt ein Formkörper ist, der ein von der Vielzahl von losen Partikeln unterschiedliches Material aufweist. Wenn also das mindestens eine Objekt ein Partikel ist, wäre es demnach denkbar, dass ein Partikelgemisch, das einzelne Partikel beinhaltend ein Material A sowie einzelne Partikel beinhaltend ein Material B aufweist, in der Aussparung vorliegt. Der nach dem Beschichtungsprozess nicht verfestigte Teil des Partikelgemisches entspräche dann dem zweiten Anteil von losen Partikeln. Wie zuvor beschrieben, kann der Schritt des zumindest teilweisen Entfernens des zweiten Anteils der losen Partikel selektiv ausgeführt werden. Das heißt, es kann beispielsweise ein selektives Entfernen einzelner Partikel aus dem Partikelgemisch stattfinden, und zwar derart, dass aus dem Partikelgemisch nur einzelne ein bestimmtes Material beinhaltende Partikel (z.B. nur einzelne Partikel beinhaltend Material A) entfernt werden, während hingegen einzelne Partikel, die das jeweils andere Material beinhalten (z.B. einzelne Partikel beinhaltend Material B) in der Aussparung übrig bleiben. Wenn das mindestens eine Objekt ein Formkörper ist, wäre es denkbar, dass der Formkörper gemeinsam mit der Vielzahl loser Partikel in der Aussparung vorliegt. Nach dem zumindest teilweisen Entfernen des zweiten Anteils der Partikel bleibt, neben eventuell nicht entfernten Partikeln, der Formkörper in dem Hohlraum zurück. Wenn die Partikel vollständig entfernt wurden, bleibt der Formkörper alleinig in dem Hohlraum zurück. Der in dem Hohlraum eingeschlossene Formkörper kann sich, sofern er kleiner als der Hohlraum ist, beispielsweise frei darin bewegen.

Das mindestens eine Objekt kann beispielsweise eine magnetische Eigenschaft aufweisen. Das heißt, das Objekt (Partikel oder Formkörper) kann ein magnetisches Material aufweisen oder aus einem magnetischen Material bestehen. Das Objekt kann beispielsweise ein magnetischer Formkörper sein, der nach einem Entfernen der unverfestigten losen Partikel alleinig in dem Hohlraum zurückbleibt. Somit erhält man sozusagen einen in dem Hohlraum eingeschlossenen Magneten, der sich in dem Hohlraum beispielsweise frei bewegen kann. Dies kann beispielsweise genutzt werden, um Lageänderungen zu detektieren.

Gemäß einer Ausführungsform des erfindungsgemäßen Verfahrens kann das mindestens eine Objekt in einem demagnetisierten Zustand in die Aussparung eingebracht werden und vor oder nach dem zumindest teilweisen Entfernen des zweiten Anteils der Partikel magnetisiert werden. Dies bietet sich beispielsweise an, wenn magnetische Eigenschaften des Objekts vor dem Entfernen der unverfestigten losen Partikel unerwünscht sind oder wenn anschließend noch Prozesse bei Temperaturen erforderlich sind, die die magnetischen Eigenschaften zu stark beeinflussen würden.

Es ist außerdem denkbar, dass eine Magnetfeldsensorik an dem Substrat angeordnet wird, um mittels der Magnetfeldsensorik das Magnetfeld des mindestens einen Objekts zu bestimmen. Es können beispielsweise bekannte Magnetfeldsensoren zum Einsatz kommen, die "on chip" in dem Substrat ausgebildet werden. Ein solcher Magnetfeldsensor ist somit unmittelbar an dem Substrat angeordnet. Es ist aber auch denkbar, das ein Magnetfeldsensor an einem separaten Träger angeordnet ist, und der Träger wiederum mit dem Substrat verbunden wird. Ein solcher Magnetfeldsensor ist somit mittelbar, d.h. mittels des separaten Trägers, an dem Substrat angeordnet.

Gemäß weiterer denkbarer Ausgestaltungen des erfindungsgemäßen Verfahrens kann eine mit dem mindestens einen Objekt wechselwirkende Spule an dem Substrat angeordnet werden, um mittels einer Relativbewegung zwischen der Spule und dem mindestens einen Objekt eine sich in der Spule einstellende Induktionsspannung zu erzeugen, oder um mittels eines durch die Spule fließenden elektrischen Stroms eine magnetische Induktionskraft auf das mindestens eine Objekt auszuüben. Die Spule kann beispielsweise in das Substrat integriert sein. Eine solche Spule ist somit unmittelbar an dem Substrat angeordnet. Es ist aber auch denkbar, dass eine externe Spule, oder ein externes Spulenarray, an dem Substrat angeordnet wird. Eine solche Spule ist somit mittelbar, z.B. mittels eines separaten Trägers, an dem Substrat angeordnet. Es ist ebenso denkbar, dass die Spule an der weiteren Schicht, insbesondere Passivierungsschicht, angeordnet ist. Auch in diesem Fall wäre die Spule mittelbar, d.h. über die Passivierungsschicht, an dem Substrat angeordnet.

Es ist vorstellbar, dass in der der porösen Struktur gegenüber liegenden Seite des Substrats eine Öffnung in das Substrat eingebracht wird, die eine Fluidverbindung zwischen der Aussparung bzw. dem Hohlraum und der Umgebung bereitstellt. So kann beispielsweise ein Fluideinlass bzw. ein Fluidauslass bereitgestellt werden. Dies ist insbesondere zum Herstellen von durchströmten Systemen, z.B. zum Messen von Fluidströmen, vorteilhaft. Beispielsweise kann die poröse Struktur als ein Fluideinlass dienen und die Öffnung kann als Fluidauslass dienen, oder anders herum.

Hierbei ist es vorstellbar, dass der Querschnitt der Öffnung kleiner ist als ein nach dem zumindest teilweisen Entfernen des zweiten Anteils der Partikel in der Aussparung verbleibendes Objekt, sodass die Öffnung mittels des Objekts verschließbar ist, um die Fluidverbindung zur Umgebung zu unterbinden. Das Objekt, z.B. ein Formkörper, kann als ein die Öffnung verschließender Deckel bzw. Ventilteller fungieren. Das Objekt kann die Öffnung verschließen und die Öffnung bei Bedarf wieder freigeben.

Allgemein kann das Bereitstellen des Substrats ein Bereitstellen eines Substrats mit einer kreisförmigen Aussparung und/oder einer langgestreckten Aussparung beinhalten. Die in dem Substrat vorgesehene Aussparung kann also beispielsweise ein langgestreckter (z.B. gefräster oder geätzter) Graben, eine Bohrung oder ein Sackloch sein. Die Aussparung kann auch in Form eines Donuts bzw. eines Toroids (ringförmiger Graben) ausgebildet sein.

Die Erfindung betrifft ferner eine Vorrichtung mit einem Substrat, das eine Aussparung aufweist, und einer porösen Struktur, die derart in oder an der Aussparung angeordnet ist, dass zwischen der Aussparung und der porösen Struktur ein Hohlraum bestehen bleibt. Das heißt, der Boden und die den Boden umgebenden Seitenwände der Aussparung bilden zusammen mit der porösen Struktur den Hohlraum. Die poröse Struktur ist dem Boden der Aussparung gegenüberliegend angeordnet. Die poröse Struktur kann beispielsweise oberhalb der Öffnung der Aussparung, d.h. auf dem Substrat, oder auch ein Stück weit innerhalb der Aussparung angeordnet sein. Die poröse Struktur weist eine Vielzahl von Partikeln auf, die mittels einer Beschichtung miteinander verbunden und zu der porösen Struktur verfestigt sind. Dies unterscheidet die erfindungsgemäße poröse Struktur von anderen herkömmlichen porösen Strukturen, was beispielsweise in einem Schnittbild unter dem Mikroskop nachvollziehbar ist. Der von der Aussparung und der porösen Struktur gebildete Hohlraum ist zu weniger als 90% seines Volumens mit losen Partikeln gefüllt. Die poröse Struktur ist eine freitragende Struktur, die sich nicht auf den losen Partikeln abstützt. Die freitragende poröse Struktur kann beispielsweise eine freitragende poröse Membran sein. Die Erfindung schafft somit eine Vorrichtung mit einem Hohlraum, der eine freitragende poröse Struktur aufweist, bzw. einen Hohlraum, der mittels einer freitragenden porösen Struktur abgedeckt ist. Der Hohlraum kann zu 90% seines Volumens, oder weniger, mit Partikeln befüllt sein, wobei zumindest ein Teil dieser Partikel dasselbe Material aufweisen kann wie die in der porösen Struktur enthaltenen Partikel. Dadurch, dass nicht der gesamte Hohlraum vollständig mit Partikeln ausgefüllt ist, können sich die Partikel innerhalb des Hohlraums relativ frei bewegen. Es wird also ein Hohlraum geschaffen, der nicht vollständig mit losen Partikeln gefüllt ist und gleichzeitig eine poröse Struktur aufweist, um hierdurch eine fluidische Kommunikation des Hohlraums mit der Umgebung zu ermöglichen. Die Partikel können auch in Form eines Partikelgemisches aus Partikeln unterschiedlicher Materialien vorliegen.

Gemäß einer Ausführungsform kann der Hohlraum zu weniger als 75%, oder zu weniger als 50%, oder zu weniger als 10% seines Volumens mit den losen Partikeln gefüllt sein. Ebenso wäre es denkbar, dass der Hohlraum keine dieser losen Partikel aufweist. Hier wäre also eine Vorrichtung mit einem Hohlraum geschaffen, der im Wesentlichen frei von den vorgenannten Partikeln ist.

Es ist denkbar, dass die in dem Hohlraum befindlichen losen Partikel ein anderes Material aufweisen als die zu der porösen Struktur verfestigten Partikel. Dies wäre beispielsweise von Vorteil, wenn die in dem Hohlraum befindlichen losen Partikel mit einem Ätzmittel entfernt werden. Dann wäre es sinnvoll, dass die losen Partikel ein Material aufweisen, das durch das Ätzmittel angreifbar ist, während die zu der porösen Struktur verfestigten Partikel ein Material aufweisen, das nicht durch das Ätzmittel angegriffen wird, um die poröse Struktur beizubehalten.

Gemäß einer Ausführungsform kann in dem Hohlraum mindestens ein Objekt angeordnet sein, wobei das mindestens eine Objekt ein Partikel ist, das ein Material aufweist, das sich von dem Material der in dem Hohlraum befindlichen losen Partikeln unterscheidet, oder wobei das mindestens eine Objekt ein Formkörper ist, der ein Material aufweist, das sich von dem Material der in dem Hohlraum befindlichen losen Partikeln unterscheidet. Wenn also in dem Hohlraum noch einige der zuvor erwähnten losen Partikel vorhanden sind, kann zusätzlich zu diesen losen Partikeln das mindestens eine Objekt in dem Hohlraum vorgesehen sein. Es wäre aber auch denkbar, dass bei vollständiger Entfernung aller dieser losen Partikel, nur das mindestens eine Objekt (oder auch mehrere derartiger Objekte), das ein anderes Material als die losen Partikel aufweist, in dem Hohlraum vorhanden ist. Das mindestens eine Objekt kann ein Partikel oder aber auch ein Formkörper sein. Es wäre also denkbar, dass in dem Hohlraum ein oder mehrere Partikel eines anderen Materials (im Vergleich zu den vorgenannten losen Partikeln) bzw. ein aus einem anderen Material bestehender Formkörper in dem Hohlraum eingeschlossen sind. Ein Formkörper ist vorzugsweise ein dreidimensionaler Formkörper. Er kann ein aus mehreren Partikeln zusammengesetzter Formkörper, oder ein nach herkömmlichen Verfahren, z.B. Spritzgießen, Sintern und dergleichen, hergestellter Formkörper sein.

Es ist vorstellbar, dass das mindestens eine Objekt eine magnetische Eigenschaft aufweist. Das mindestens eine Objekt kann beispielsweise eine hartmagnetische oder eine weichmagnetische Eigenschaft aufweisen. Das mindestens eine Objekt kann ein beliebiger Magnet sein. Das mindestens eine magnetische Objekt kann kleiner sein als das Volumen der Aussparung, so dass sich das magnetische Objekt in der Aussparung frei bewegen kann. Dies bringt den Vorteil, dass beispielsweise bei einer Lageänderung, bei einer Positionsänderung oder bei einer Beschleunigung der Vorrichtung das mindestens eine Objekt in der Aussparung bewegt. Mittels geeigneter Sensorik kann diese Bewegung des magnetischen Objekts in der Aussparung ermittelt werden.

Demnach ist es vorteilhaft, wenn eine Magnetfeldsensorik an dem Substrat angeordnet ist, wobei die Magnetfeldsensorik ausgebildet ist, um das Magnetfeld des mindestens einen Objekts zu bestimmen. Die Magnetfeldsensorik kann beispielsweise in das Substrat integriert sein. Eine solche Magnetfeldsensorik ist somit unmittelbar an dem Substrat angeordnet. Es ist aber auch denkbar, dass eine externe Magnetfeldsensorik an dem Substrat angeordnet wird. Eine solche externe Magnetfeldsensorik ist somit mittelbar, z.B. mittels eines separaten Trägers, an dem Substrat angeordnet. Es ist ebenso denkbar, dass die Magnetfeldsensorik an der weiteren Schicht, insbesondere Passivierungsschicht, angeordnet ist. Auch in diesem Fall wäre die Magnetfeldsensorik mittelbar, d.h. über die Passivierungsschicht, an dem Substrat angeordnet.

Auch wäre es denkbar, dass eine mit dem mindestens einen Objekt wechselwirkende Spule an dem Substrat angeordnet ist, die ausgebildet ist, um mittels einer Relativbewegung zwischen der Spule und dem mindestens einen Objekt eine sich in der Spule einstellende Induktionsspannung zu erzeugen, oder um mittels eines durch die Spule fließenden elektrischen Stroms eine magnetische Induktionskraft auf das mindestens eine Objekt auszuüben. Die Spule kann beispielsweise in das Substrat integriert sein. Eine solche Spule ist somit unmittelbar an dem Substrat angeordnet. Es ist aber auch denkbar, dass eine externe Spule, oder ein externes Spulenarray, an dem Substrat angeordnet wird. Eine solche Spule ist somit mittelbar, z.B. über einen separaten Träger, an dem Substrat angeordnet. Es ist ebenso denkbar, dass die Spule an der weiteren Schicht, insbesondere Passivierungsschicht, angeordnet ist. Auch in diesem Fall wäre die Spule mittelbar, d.h. über die Passivierungsschicht, an dem Substrat angeordnet.

Es ist vorstellbar, dass die poröse Struktur eine Vielzahl von Partikeln mit magnetischer Eigenschaft aufweist. Somit würde die poröse Struktur eine magnetische Eigenschaft aufweisen. Wenn beispielsweise ein oder mehrere Objekte in dem Hohlraum vorhanden sind, die ebenfalls eine magnetische Eigenschaft aufweisen, dann könnten diese mit der magnetischen porösen Struktur in Wechselwirkung treten. Somit könnte beispielsweise die poröse Struktur (z.B. mittels einer Spule) derart magnetisiert werden, dass sie das magnetische Objekt anzieht und festhält. Somit könnte beispielsweise eine Magnetventil-Funktionalität mittels der erfindungsgemäßen Vorrichtung bereitgestellt werden.

Es ist denkbar, dass eine Beschichtung, insbesondere eine Passivierungsschicht, auf die poröse Struktur aufgebracht wird, sodass sich die Beschichtung zumindest abschnittsweise über das Substrat und/oder zumindest abschnittsweise über die poröse Struktur erstreckt. Eine solche zusätzliche Schicht könnte beispielsweise dazu dienen, die poröse Struktur zu versiegeln, um diese somit gegen das Eindringen von z.B. Schmutz, Staub, Feuchtigkeit und dergleichen zu schützen. Die zusätzliche Schicht kann sich beispielsweise auch nur teilweise über die poröse Struktur hinweg erstrecken, so dass ein Teil der porösen Struktur weiterhin der Umgebung ausgesetzt ist.

Es wäre vorstellbar, dass die Beschichtung die poröse Struktur vollständig bedeckt und die poröse Struktur fluiddicht versiegelt. Eine solche Beschichtung kann beispielsweise mittels einer Epoxymatrix, mittels Silikon oder mittels eines Gummiwerkstoffs erfolgen.

Gemäß einer Ausführungsform kann in der der porösen Struktur gegenüber liegenden Seite des Substrats eine Öffnung in dem Substrat ausgebildet sein, die eine Fluidverbindung zwischen der Aussparung und der Umgebung bereitstellt. Diese Öffnung kann somit also dazu dienen, einen Fluideinlass bzw. einen Fluidauslass bereitzustellen, so dass die Aussparung fluidisch mit der Umgebung kommunizieren kann.

Hierbei ist es denkbar, dass der Querschnitt der Öffnung kleiner ist als ein in dem Hohlraum befindliches Objekt, sodass die Öffnung mittels des Objekts verschließbar ist, um die Fluidverbindung zur Umgebung zu unterbinden. Es kann also mittels des Objekts eine Ventilfunktionalität bereitgestellt werden, wobei das Objekt selektiv die Öffnung freigeben bzw. verschließen kann.

Das Substrat kann eine kreisförmige Aussparung und/oder einer langgestreckte Aussparung aufweisen. Die in dem Substrat vorgesehene Aussparung kann also beispielsweise ein langgestreckter (z.B. gefräster oder geätzter) Graben, eine Bohrung oder ein Sackloch sein. Die Aussparung kann auch in Form eines Donuts bzw. eines Toroids (ringförmiger Graben) ausgebildet sein.

Ausführungsbeispiele der Erfindung sind in der Zeichnung dargestellt und werden nachstehend erläutert. Es zeigen:
- Fig. 1a bis 1d: eine schematische Darstellung des erfindungsgemäßen Verfahrens,
- Fig. 1e: eine schematische Darstellung eines optionalen Schritts des erfindungsgemäßen Verfahrens,
- Fig. 2a und 2b: eine schematische Darstellung mit in der Vielzahl loser Partikel vorhandener Formkörpern,
- Fig. 3a und 3b: eine schematische Darstellung zur Verkippung einer Vorrichtung mit einem in dem Hohlraum eingeschlossenen Formkörper,
- Fig. 4a und 4b: die Darstellung der Magnetfeldstärke einer NdFeB-Kugel in Abhängigkeit von deren Radius,
- Fig. 5a und 5b: eine schematische Darstellung des Bereitstellens einer Magnetfeldsensorik,
- Fig. 6a bis 6c: eine schematische Darstellung des Bereitstellens einer oder mehrerer Spulen bzw. Spulenarrays,
- Fig. 7a bis 7c: eine schematische Darstellung von durchströmten Systemen, und
- Fig. 8a bis 8d: Ausführungsformen verschiedener Ausgestaltungen der in dem Substrat vorgesehenen Aussparung.

Bevor nachfolgend Ausführungsbeispiele der vorliegenden Erfindung im Detail anhand der Zeichnung näher erläutert werden, wird darauf hingewiesen, dass identische, funktionsgleiche oder gleichwirkende Elemente, Objekte und/oder Strukturen in den unterschiedlichen Figuren mit den gleichen Bezugszeichen versehen sind, so dass die in unterschiedlichen Ausführungsbeispielen dargestellte Beschreibung dieser Elemente untereinander austauschbar ist bzw. aufeinander angewendet werden kann.

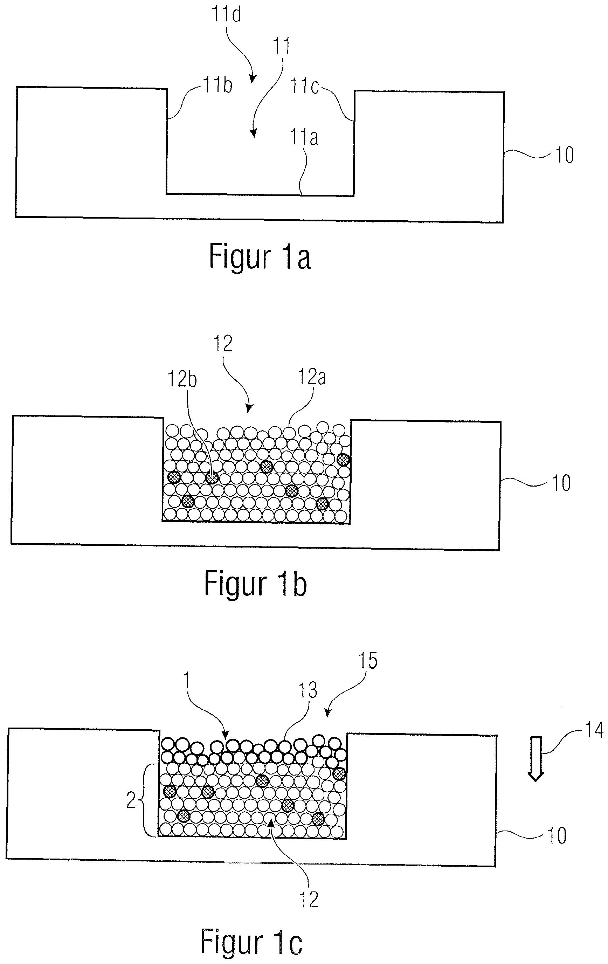

Die Figuren 1a bis 1d zeigen eine Ausführungsform eines erfindungsgemäßen Verfahrens. In Figur 1a wird zunächst ein Substrat 10 bereitgestellt. Das Substrat 10 weist eine Aussparung 11 auf. Die Aussparung 11 weist einen Boden 11a, seitliche Wände 11b, 11c und eine zur Umgebung hin weisende Öffnung 11d auf.

In Figur 1b ist gezeigt, dass eine Vielzahl loser Partikel 12 in die Aussparung 11 eingebracht wird. Dies kann beispielsweise mittels Hineinschütten von als losem Schüttgut vorliegenden Partikeln 12 in die Aussparung 11, oder aber auch durch Rakeln und dergleichen erfolgen. Die Vielzahl loser Partikel 12 kann aus lauter gleichartigen Partikeln bestehen. Die Vielzahl loser Partikel kann aber auch unterschiedliche Partikel aufweisen.

So weist beispielsweise die in den Figuren 1b und 1c abgebildete Vielzahl loser Partikel 12 eine erste Art von losen Partikeln 12a und eine zweite Art von losen Partikeln 12b auf. Die Anzahl der unterschiedlichen Partikel kann gleich sein. Wie in den Figuren gezeigt ist, kann aber auch die Anzahl der ersten Art von losen Partikeln 12a die Anzahl der zweiten Art von losen Partikeln 12b deutlich übersteigen, z.B. um einen Faktor 20 oder mehr.

Ein erster Anteil 1 der losen Partikel 12 wird unter Verwendung eines Beschichtungsprozesses beschichtet. Der Beschichtungsprozess führt dazu, dass die losen Partikel 12 mit dieser Beschichtung eine Verbindung eingehen und zu einer porösen Struktur 13 verfestigt werden. Der Beschichtungsprozess kann dabei derart gesteuert werden, dass mittels geeigneter Prozessführung eine Eindringtiefe der Beschichtung von der Öffnung 11d der Aussparung 11 ausgehend entlang einer Tiefenrichtung 14 in die Aussparung 11 hinein bestimmbar ist. Das heißt, die Dicke (in Tiefenrichtung 14) der sich ausbildenden porösen Struktur 13 kann definiert bzw. bestimmt werden.

Erfindungsgemäß wird hierbei die Eindringtiefe des Beschichtungsprozesses in die Aussparung 11 so eingestellt, dass ein zweiter Anteil 2 der Partikel 12 nicht mittels der Beschichtung verbunden wird. Das heißt, der zweite Anteil 2 der losen Partikel 12 verfestigt sich nicht zu einer porösen Struktur sondern der zweite Anteil 2 der losen Partikel 12 bleibt weiterhin in Form loser Partikel bestehen.

Erfindungsgemäß ist der zweite Anteil 2 der losen Partikel 12 zwischen dem zu der porösen Struktur 13 verfestigten ersten Anteil 1 der losen Partikel 12 und dem Innenraum (Boden 11a, Seitenwände 11b, 11c) der Aussparung 11 angeordnet. In anderen Worten ist der zweite Anteil 2 loser Partikel 12 in einem Hohlraum, der sich zwischen der Aussparung 11 und der porösen Struktur 13 bildet, angeordnet. Anders ausgedrückt ist der zu der porösen Struktur 13 verfestigte erste Anteil 1 der Partikel 12 zwischen dem zweiten Anteil 2 der Partikel 12 und einer Umgebung 15 der Aussparung 11 angeordnet. Mit der Umgebung 15 ist die Umgebung gemeint, in der sich das Substrat 10 befindet, d.h. das Medium, das das Substrat 10 umgibt.

Wie in Figur 1c zu erkennen ist, wird der oben aufliegende Teil der in die Aussparung 11 eingebrachten Partikel 12 beschichtet, so dass sich die beschichteten Partikel 12 zu der porösen Struktur 13 verfestigen. Dadurch ergibt sich, dass nach Ausbilden der porösen Struktur 13 die darunter liegenden, nicht beschichteten und somit lose bleibenden Partikel 2 direkt an die Unterseite der porösen Struktur 13 angrenzen. Das Volumen des sich zwischen der porösen Struktur 13 und dem Innenraum 11a, 11b, 11c der Aussparung 11 ausbildenden Hohlraums 16 ist somit nahezu vollständig, d.h. zu etwa 100% seines Volumens mit losen Partikeln 12 gefüllt.

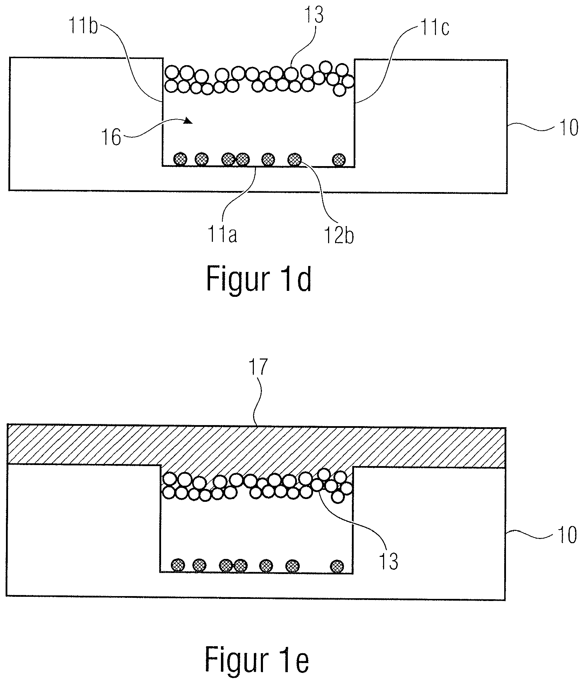

Figur 1d zeigt das Endprodukt eines weiteren Schritts des erfindungsgemäßen Verfahrens. Zwischen den Figuren 1c und 1d wurde ein Teil des zweiten Anteils 2 der Partikel 12 aus der Aussparung 11 entfernt. Im vorliegenden Ausführungsbeispiel wurden beispielsweise alle Partikel 12a der ersten Art entfernt, wobei alle Partikel 12b der zweiten Art erhalten geblieben sind. Genauso gut wäre es denkbar, dass alle Partikel 12b der zweiten Art entfernt werden, während hingegen alle Partikel 12a der ersten Art erhalten bleiben. Die Partikel 12 können, müssen aber nicht vollständig entfernt werden. Erfindungsgemäß wird der zweite Anteil 2 der Partikel 12 zumindest teilweise entfernt, d.h. der zweite Anteil der Partikel 12 wird zu mindestens 10% aus der Aussparung 11 entfernt.

In dem in Figur 1d gezeigten Beispiel ist also zu sehen, dass durch das Entfernen aller Partikel der ersten Art 12a lediglich die Partikel der zweiten Art 12b in dem Hohlraum 16 übrig bleiben. Der Hohlraum 16 bildet sich zwischen dem Boden 11a und den Seitenwänden 11b, 11c der Aussparung 11 sowie der porösen Struktur 13 aus. Die nach dem Entfernen der Partikel der ersten Art 12a übrig bleibenden Partikel der zweiten Art 12b sind sozusagen in dem Hohlraum 16 eingeschlossen.

Bei dem Beschichtungsprozess zum Erzeugen der porösen Struktur 13 kann es vorteilhaft sein, wenn die Beschichtung auf der Oberseite (die dem Boden 11a der Aussparung 11 gegenüberliegende Seite) der losen Partikel 12 aufgebracht wird. Die Beschichtung wird also auf die oben aufliegenden losen Partikel 12 aufgebracht. Der Beschichtungsprozess kann ein ALD-Prozess (ALD = Atomic Layer Deposition, dt. Atomlagenabscheidung) sein. Dieser Beschichtungsprozess ist selbstbegrenzend, d.h. der ALD-Beschichtungsprozess führt zu einer vorgegebenen Schichtdicke und nicht darüber hinaus.

Die losen Partikel 12, genauer gesagt die mit der ALD-Beschichtung in Kontakt kommenden losen Partikel 12 verfestigen sich und bilden eine verfestigte aber poröse Struktur 13 aus. Die poröse Struktur 13 ist durchlässig für bestimmte Medien, wie z.B. Luft. Demnach können die durchlässigen Medien durch die poröse Struktur 13 hindurch, d.h. entweder in die Aussparung 11 hinein oder aus der Aussparung 11 heraus, diffundieren.

Die zu der porösen Struktur 13 verfestigten Partikel 12 werden auch als ein erster Anteil 1 von Partikeln 12 bezeichnet. Die in dem Hohlraum 16 befindlichen Partikel 12 werden auch als ein zweiter Anteil 2 von Partikeln 12 bezeichnet.

Ein Teil des zweiten Anteils 2 von Partikeln 12 wird erfindungsgemäß entfernt. Der zweite Anteil 2 von Partikeln 12 kann aber auch vollständig entfernt werden (nicht dargestellt). Somit bliebe ein Hohlraum 16, der vollständig frei von Partikeln 12 ist.

Wie eingangs mit Bezug auf Figur 1c erwähnt, kann der zweite Anteil 2 von Partikeln 12 eine erste Art 12a und eine zweite Art 12b von Partikeln 12 aufweisen. Es handelt sich bei dem zweiten Anteil 2 von Partikeln 12 also um ein Partikelgemisch 12 aufweisend Partikel einer ersten Art 12a und Partikel einer zweiten Art 12b.

Mit dem erfindungsgemäßen Verfahren kann ein Teil, z.B. 10% oder mehr, der ersten Art 12a von Partikeln aus dem ursprünglich in die Aussparung 11 eingebrachten Partikelgemisch 12 entfernt werden. Somit bleiben in dem Hohlraum 16 sowohl Partikel der zweiten Art 12b als auch der nicht entfernte Teil von Partikeln der ersten Art 12a erhalten. Die erste Art 12a von Partikeln kann aber auch vollständig aus dem Partikelgemisch 12 entfernt werden, so dass lediglich Partikel der zweiten Art 12b in dem Hohlraum 16 erhalten bleiben, wie dies in Figur 1d gezeigt ist.

Der Teil des zweiten Anteils 2 von Partikeln 12 kann mittels unterschiedlicher Verfahren entfernt werden. Zum Beispiel kann ein Ätzverfahren angewandt werden, um Partikel aus dem Partikelgemisch 12 zu entfernen. Mittels eines geeigneten Ätzmittels kann beispielsweise selektiv eine bestimmte Art von Partikeln aus dem Partikelgemisch 12 weggeätzt bzw. entfernt werden.

Besteht beispielsweise das Partikelgemisch 12 aus Silizium und Siliziumoxid, könnten die Siliziumpartikel 12a in XeF₂-Gasphase bzw. die Siliziumoxidpartikel 12b in HF-Gasphase selektiv zum jeweiligen anderen und vielen weiteren Materialien geätzt werden. Es existieren noch viele andere Kombinationsmöglichkeiten. Generell werden trockenchemische Ätzprozesse bevorzugt, nasschemische jedoch nicht ausgeschlossen.

Generell kann der erste Anteil 1 der Partikel 12 ein anderes Material aufweisen als der zweite Anteil 2 der Partikel 12. Dies kann beispielsweise dadurch erreicht werden, dass zuerst lose Partikel 12, die ein erstes Material aufweisen, in die Aussparung 11 eingebracht werden. Anschließend werden lose Partikel 12, die ein zweites Material aufweisen, in die Aussparung 11 eingebracht. Das heißt, die das zweite Material aufweisenden losen Partikel 12 werden auf die das erste Material aufweisenden losen Partikel 12 aufgeschüttet. Anschließend werden die oben aufliegenden losen Partikel 12 mit dem erfindungsgemäßen Beschichtungsprozess beschichtet.

Dabei ist es vorteilhaft, wenn lediglich das Material der oben aufliegenden losen Partikel 12 für die Beschichtung reaktiv ist. Somit wird erreicht, dass lediglich die oben aufliegenden losen Partikel 12 unter Verwendung des Beschichtungsprozesses zu einer porösen Struktur 13 verhärten. Die darunterliegenden losen Partikeln 12 aus dem anderen Material verhärten hingegen nicht, selbst wenn sie mit der Beschichtung in Kontakt kommen, da das Material dieser darunterliegenden losen Partikel 12 nicht für die Beschichtung reaktiv ist.

In anderen Worten ausgedrückt kann es von Vorteil sein, wenn der verfestigte obere Bereich gemäß Figur 1c aus Partikeln 12 eines ganz bestimmten Materials C und der Verfestigungsschicht 13 besteht. Erreicht werden kann das durch ein sequentielles Einbringen der Partikel 12. Zuerst wird die Aussparung 11 mit dem Partikelgemisch 12, aufweisend Partikel 12a mit Material A sowie Partikel 12b mit Material B, vorbefüllt. Anschließend wird die Aussparung 11 in einem zweiten Befüllungsschritt mit Partikeln des Typs C aufgefüllt. Die anschließende Verfestigung im oberen Bereich wird bezüglich der Tiefe so ausgeführt, dass sich der Prozess überwiegend auf die Partikel vom Typ C beschränkt.

Figur 1e zeigt einen optionalen Verfahrensschritt des erfindungsgemäßen Verfahrens. Hier wird eine Beschichtung 17 auf die poröse Struktur 13 aufgebracht. Die Beschichtung 17 ist vorzugsweise eine Passivierungsschicht. Die Beschichtung 17 erstreckt sich in dem in Figur 1e gezeigten Ausführungsbeispiel vollständig über die poröse Struktur 13. Die Beschichtung 17 erstreckt sich in dem in Figur 1e gezeigten Ausführungsbeispiel außerdem vollständig über das Substrat 10. Die Beschichtung 17 kann die poröse Struktur 13 fluiddicht versiegeln.

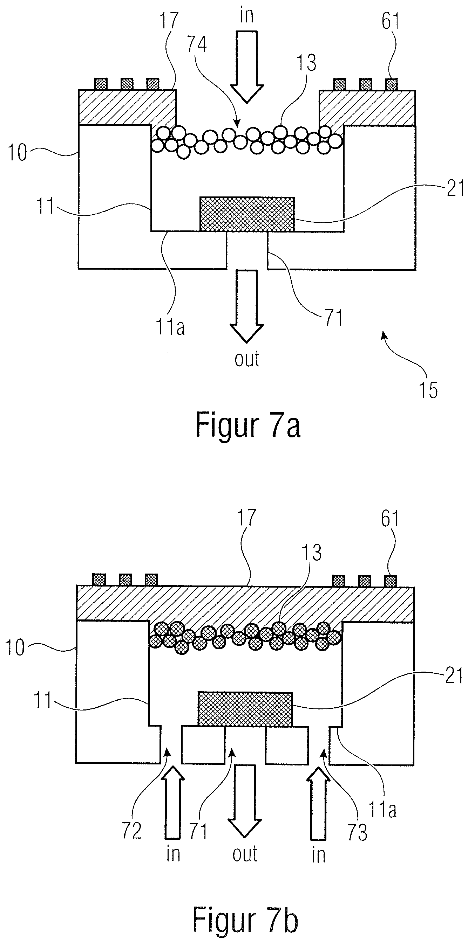

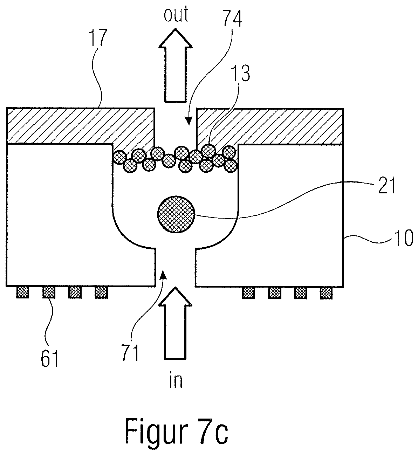

Wie nachfolgend unter Bezugnahme auf die Figuren 7a und 7c noch beschrieben wird, kann die Beschichtung 17 die poröse Struktur 13 bzw. das Substrat 10 auch lediglich teilweise bzw. abschnittsweise bedecken. Durch das Aufbringen der Beschichtung 17 auf der porösen Struktur 13 kann der Hohlraum 16 mit den Partikeln 12b verschlossen werden. Bei Bedarf kann der Hohlraum 16 unter Vakuum oder in einer bestimmten Atmosphäre verschlossen werden.

Wie eingangs erwähnt wurde, kann die Vielzahl von Partikeln12 ein Partikelgemisch sein, das Partikel 12a einer ersten Art (bzw. eines ersten Materials) und Partikel 12b einer zweiten Art (bzw. eines zweiten Materials) beinhaltet. Zusätzlich oder alternativ zu den Partikeln 12b der zweiten Art kann ein Formkörper 21, 22 in die Aussparung 11 eingebracht werden.

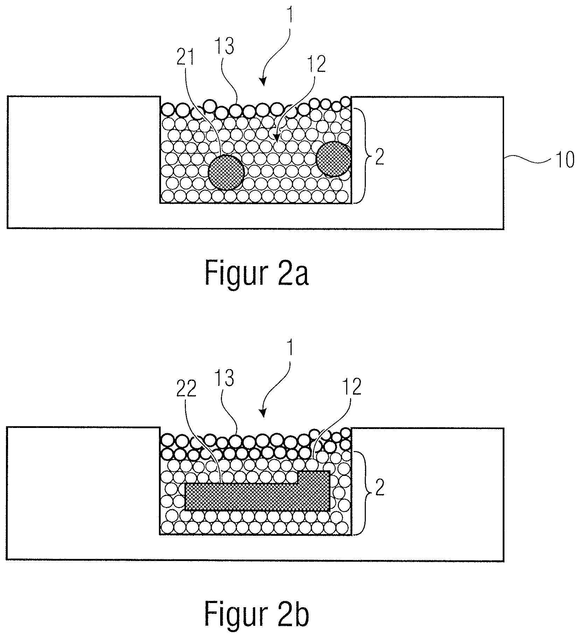

In Figur 2a ist beispielsweise ein kugelförmiger Formkörper 21 abgebildet, der zusammen mit der Vielzahl loser Partikel 12 in der Aussparung 11 eingebracht ist. Genauer gesagt befindet sich der Formkörper 21 zwischen den losen Partikeln 12 des zweiten Anteils 2 loser Partikel 12, während der erste Anteil 1 der Partikel 12 zu einer porösen Struktur 13 verfestigt ist. In Figur 2b ist beispielsweise ein L-förmiger Formkörper 22 abgebildet, der zusammen mit der Vielzahl loser Partikel 12 in der Aussparung 11 eingebracht ist.

Vorzugsweise weist der Formkörper 21, 22 ein anderes Material auf als die Vielzahl der in der Aussparung 11 beinhalteten losen Partikel 12. Beispielsweise kann die Vielzahl loser Partikel 12 ein Material aufweisen, das mittels eines Ätzmittels aus der Aussparung 11 entfernbar ist, während das Material des Formkörpers 21, 22 nicht auf das Ätzmittel reagiert. So kann selektiv die Vielzahl der Partikel 12 aus der Aussparung entfernt werden, wobei der bzw. die Formkörper 21, 22 in der Aussparung 11 bleiben.

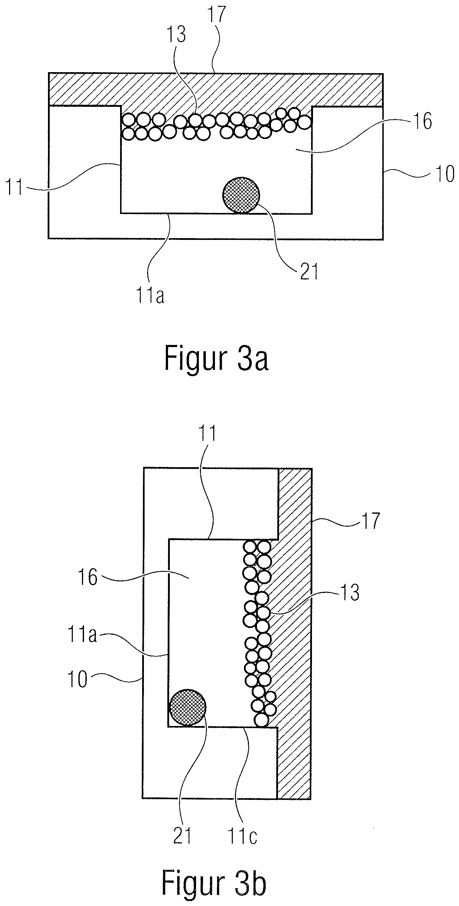

Die Figuren 3a und 3b zeigen eine weitere Ausführungsform gemäß welcher der zweite Anteil 2 der Partikel 12 unterhalb der porösen Struktur 13 entfernt wurde. Es bleibt lediglich der zuvor mit Bezug auf Figur 2a beschriebene kugelförmige Formkörper 21 übrig. Der Formkörper 21 befindet sich in dem Hohlraum 16, der sich aufgrund des Entfernens des zweiten Anteils 2 der losen Partikel 12 ergibt. Der Formkörper 21 liegt auf dem Boden 11a der Aussparung 11 auf. Außerdem ist die poröse Struktur 13 mit einer Beschichtung 17 auf ihrer Außenseite versiegelt.

Figur 3b zeigt die Vorrichtung aus Figur 3a, jedoch um 90° nach rechts gekippt. Dementsprechend fällt der Formkörper 21 nach unten, so dass er zumindest abschnittsweise auf der rechten Seitenwand 11c der Aussparung 11 aufliegt. Außerdem kann der Formkörper 21 zumindest abschnittsweise auf dem Boden 11a der Aussparung 11 anliegen.

In derartigen Ausführungsformen kann es sinnvoll sein, die Innenseiten der Aussparung 11 (d.h. den Boden 11a und/oder die Seitenwände 11b, 11c) zumindest abschnittsweise zu beschichten, um die Adhäsionskräfte bzw. Reibungskräfte der Innenseiten 11a, 11b, 11c der Aussparung 11 gegenüber dem Formkörper 21 zu verringern. Ebenso kann es sinnvoll sein, eine Beschichtung auf mindestens einer Innenseite 11a, 11b, 11b der Aussparung 11 aufzubringen, die eine von dem Formkörper 21 ausgehende Strahlung verringert bzw. abschirmt. Hierbei kann es sich beispielsweise um eine Metallschicht handeln.

Es kann also beispielsweise von Vorteil sein, die Innenseiten 11a, 11b, 11c der Aussparung 11 vor dem Einbringen der Partikel 12 und Formkörper 21, 22 gemäß Figur 1b mit einer Schicht auszukleiden, abgeschieden z.B. mittels Sputtern, CVD und anderer in der Halbleitertechnologie etablierter Verfahren. Das kann z.B. eine Schicht sein, die das Adhäsions- bzw. Reibungsverhalten der frei beweglichen Partikel 12 oder Formkörper 21, 22 an den Innenseiten 11a, 11b, 11c der Kavität 11 beeinflusst. Des Weiteren kann dies eine metallische Schicht sein, um diese elektromagnetisch abzuschirmen. Es kann auch eine Abfolge mehrerer Schichten aufgebracht werden, die dieselbe oder unterschiedliche Funktionalitäten haben.

Wie soeben mit Bezug auf die Figuren 3a und 3b erläutert wurde, kann ein in dem Hohlraum 16 befindliches Objekt (Partikel 12b und/oder Formkörper 21, 22) in Abhängigkeit der Lage des Substrats 10 eine bestimmte Position in dem Hohlraum 16 einnehmen. Eine Lageänderung kann gut ermittelt bzw. bestimmt werden, wenn es sich bei dem in dem Hohlraum 16 befindlichen Objekt 12b, 21, 22 um ein Objekt mit magnetischer Eigenschaft handelt.

Gemäß einer Ausführungsform des erfindungsgemäßen Verfahrens wird ein Objekt (Partikel 12b und/oder Formkörper 21, 22) mitsamt der losen Partikel 12a in die Aussparung eingebracht, wie in den Figuren 1b, 1c und 2a, 2b gezeigt. Vorzugsweise wird das Objekt 12b, 21, 22 nach dem Entfernen des Teils des zweiten Anteils der losen Partikel 12a (Figuren 1d, 3a, 3b) aufmagnetisiert.

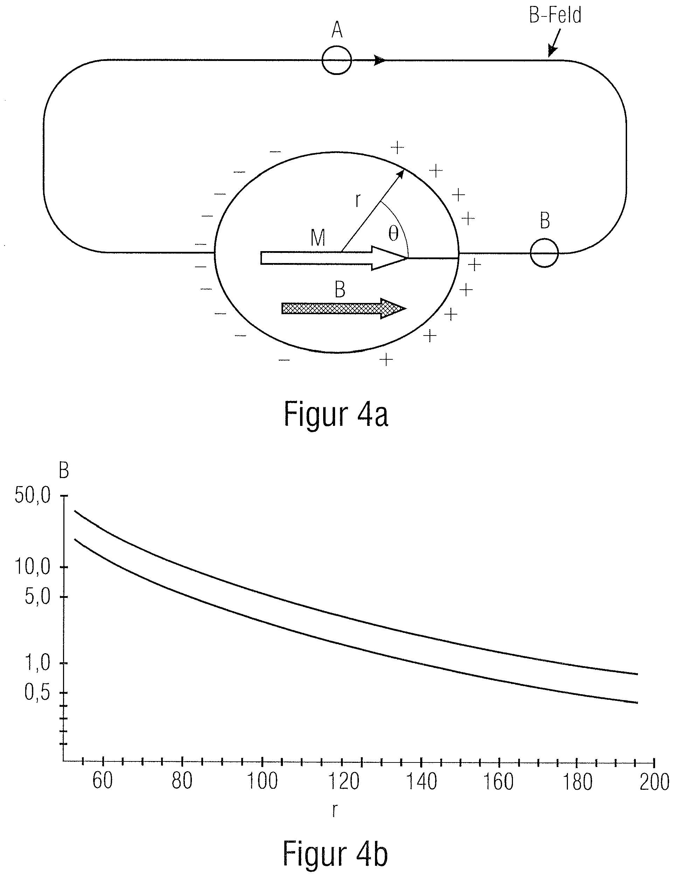

Die Partikel 12b oder Formkörper 21, 22 können aus beliebigen hartmagnetischen Materialien bestehen. Als Detektoren kommen alle bekannten Magnetfeldsensoren, wie z. B. Hall-, AMR-oder GMR-Sensoren, sowie Spulen (Induktivitäten) in Betracht. Von besonderem Interesse sind Arrays aus magnetfeldempfindlichen Bauelementen, die eine hohe Positionsauflösung erlauben und unter Verwendung CMOS-kompatibler Pozesse hergestellt werden können, wie z. B. die sogenannten split-drain MOSFET (MAGFET) [1]. In [2] wird berichtet, dass sich die C-V-Kennlinie eines "n-channel enhancement type MOSFET" unter dem Einfluss eines magnetischen Feldes von 70 mT um mehrere Prozent ändert. Wie in den Figuren 4a und 4b gezeigt ist, lassen sich vergleichbare Feldstärken bereits durch eine NdFeB-Kugel mit nur 25 µm Radius erzeugen. So wären Transistoren im Abstand von 50 µm vom Mittelpunkt der Kugel z.B. Feldern von 20 bis 50 mT ausgesetzt. Im Vergleich dazu, das Magnetfeld der Erde liegt unterhalb von 0,5 mT. Die Figuren 4a und 4b visualisieren die beschriebene magnetische Feldstärke B in Abhängigkeit vom Abstand (Radius) r für das Magnetfeld einer NdFeB-Kugel mit 25 µm Radius.

Unter diesen vorgenannten Erkenntnissen soll nochmals auf die Figuren 3a und 3b verwiesen werden. Befinden sich frei bewegliche magnetische Partikel 12b oder Formkörper 21, 22 im abgeschlossenen, z.B. hermetisch versiegelten Hohlraum 16, und wird diese Anordnung mit einem oder mehreren geeigneten Detektoren kombiniert, die in einem hinreichend geringen Abstand zum Hohlraum 16 angeordnet sind, können Lageänderungen der magnetischen Partikel 12b oder Formkörper 21, 22 innerhalb des Hohlraums 16 festgestellt werden, hervorgerufen z. B. durch Schwingungen, Erschütterungen oder Verkippungen. Letzteres ist in Figur 3b beispielhaft illustriert. Die im Hohlraum 16 eingeschlossenen Partikel 12b oder Formkörper 21, 22 können durchaus sehr empfindlich auf Bewegungen aller Art reagieren, wenn der Hohl raum 16 vorzugsweise hermetisch unter Ausschluss von Feuchtigkeit bzw. unter Vakuum versiegelt und insbesondere wenn er zudem noch auf der Innenseite 11a, 11b, 11c mit einer Beschichtung zur Verringerung der Adhäsion versehen ist. Von Vorteil ist dabei, dass die Partikel 12b oder Formkörper 21, 22 frei beweglich und nicht etwa federnd gelagert sind, da dadurch eine weitgehend frequenzunabhängige Empfindlichkeit gewährleistet werden kann. Zudem sind Baugrößen weit unterhalb von 1 mm³ und damit auch sehr kostengünstige Komponenten möglich.

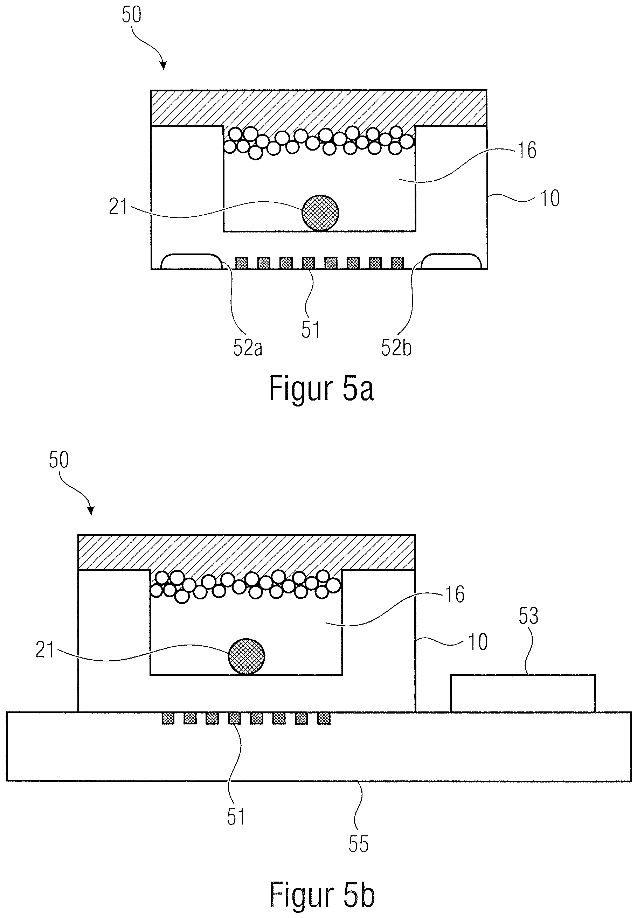

Die Figuren 5a und 5b zeigen Ausführungsbeispiele eines Bewegungssensors 50 mit einem einzelnen, kugelförmigen magnetischen Formkörper 21. Hier ist eine Magnetfeldsensorik 51 an dem Substrat 10 angeordnet, die ausgebildet ist, um das Magnetfeld des Objekts (Partikel 12b und/oder Formkörper 21, 22) zu bestimmen.

In Figur 5a ist die Magnetfeldsensorik 51 on-chip realisiert, d.h. die Magnetfeldsensorik 51 ist in dem Substrat 10 ausgebildet und zwar so, dass die Magnetfeldsensorik 51 einer Innenseite 11a, 11b, 11c der Aussparung 11 gegenüberliegend angeordnet ist. Außerdem ist eine Auswerteschaltung 52a, 52b on-chip, d.h. in dem Substrat 10 ausgebildet. Die Magnetfeldsensorik 51 ist somit also unmittelbar an dem Substrat 10 angeordnet.

In Figur 5b ist die Magnetfeldsensorik 51 auf einem separaten Träger 55 angeordnet. Außerdem ist eine Auswerteschaltung 53 auf dem separaten Träger 55 angeordnet. Der separate Träger 55 ist an dem Substrat 10 angeordnet, und zwar so, dass die Magnetfeldsensorik 51 einer Innenseite 11a, 11b, 11c der Aussparung 11 gegenüberliegend angeordnet ist. Die Magnetfeldsensorik 51 ist somit also mittelbar, d.h. mittels des separaten Trägers 55, an dem Substrat 10 angeordnet.

Die Magnetfeldsensorik 51 kann z.B. ein Magnet-Detektor bzw. Detektorarray sein. In Figur 5a sind also der Detektor (Detektorarray) 51 und die Auswerteelektronik 52a, 52b on-chip integriert. In Figur 5b ist der Sensorchip 10 auf einem separaten Träger 55 montiert, der auch den Detektor (Detektorarray) 51 und die Auswerteschaltung 53 trägt. Der Detektor (Detektorarray) 55 und die Auswerteschaltung 53 sind hier hybrid auf dem separaten Träger 55 montiert.

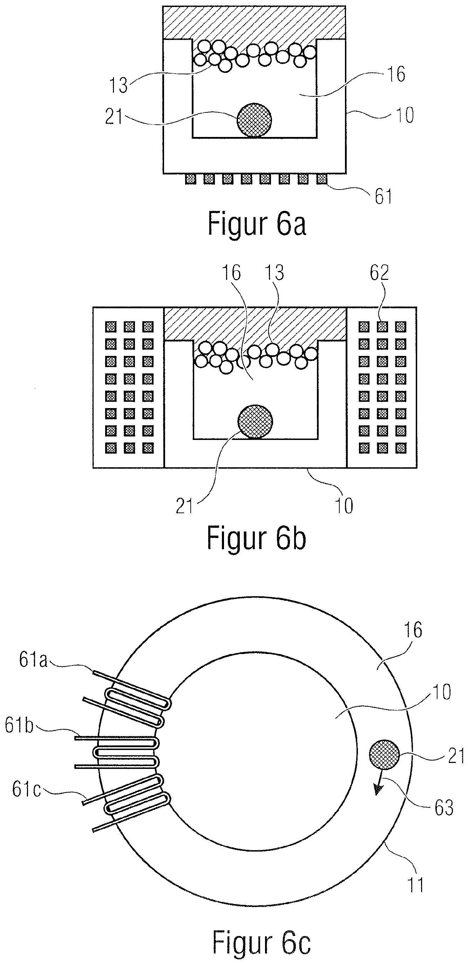

Die Figuren 6a, 6b und 6c zeigen eine weitere Ausführungsform, wobei anstelle der zuvor beschriebenen Magnetdetektoren 51 eine oder mehrere Spulen bzw. ein oder mehrere Spulenarrays zum Einsatz kommen.

In Figur 6a ist ein weiterer Verfahrensschritt des erfindungsgemäßen Verfahrens abgebildet, gemäß dem eine mit dem Objekt (Partikel 12b und/oder Formkörper 21, 22) wechselwirkende Spule 61 an dem Substrat 10 angeordnet ist. Mittels einer Relativbewegung zwischen der Spule 61 und dem magnetischen Objekt (Partikel 12b und/oder Formkörper 21, 22) wird eine sich in der Spule 61 einstellende Induktionsspannung erzeugt. Die Spule 61 kann on-chip, d.h. in dem Substrat 10 ausgebildet werden, d.h. die Spule 61 ist unmittelbar an dem Substrat 10 angeordnet.

In Figur 6b ist ein ähnliches System abgebildet, wobei anstelle der on-chip integrierten Spule 61 (Figur 6a) eine hybrid montierte externe Spule 62 bzw. ein hybrid montiertes externes Spulenarray 62 an dem Substrat 10 angeordnet ist. Die Spule 62 bzw. das Spulenarray 62 ist also mittelbar an dem Substrat 10 angeordnet.

Die in den Figuren 6a und 6b dargestellten Vorrichtungen könnten im Sinne von Bewegungssensoren beispielsweise als nichtresonante Energy-Harvester genutzt werden, wenn also anstelle des Detektors 51 oder Detektorarrays 51 (Figuren 5a, 5b) eine Spule 61 oder ein Spulenarray 61 tritt. Die zwei Grundausführungen mit einseitig integrierter, planarer bzw. hybrid montierter, dreidimensionaler Spule 61 sind in den Figuren 6a und 6b schematisch dargestellt.

Eine weitere mögliche Ausführungsform ist in Figur 6c dargestellt, die ein Draufsicht auf ein Substrat 10 mit einer Aussparung 11 zeigt. Ein magnetischer kugelförmiger Formkörper (z.B. Permanentmagnet) 21 kann sich unter dem Einfluss eines geeigneten Anregungssignals in einer ringförmigen Aussparung bzw. Hohlraum 16 im Kreis bewegen (siehe Pfeil 63). Dabei passiert er nacheinander eine Vielzahl entlang der ringförmigen Aussparung 11 angeordneter Spulen 61a, 61b, 61c, in denen dadurch Spannungspulse induziert werden.

Generell kann die erfindungsgemäße Vorrichtung als ein MEMS-Bauelement (Mikrosystem) ausgeführt sein. Da in einem MEMS-Bauelement sehr geringe Abstände von wenigen Mikrometern zwischen Spule 61a, 61b, 61c und Magnet 21 realisiert werden können, ist die zu erwartende magnetische Flussdichte größer als in einem vergleichbaren makroskopischen Aufbau. Die Querschnittsfläche A der Spule und die Wicklungszahl N sind jedoch kleiner als bei einem makroskopischen Aufbau.

Um dennoch vergleichbare Spannungen zu generieren müsste die magnetische Flussdichte in einem MEMS-Bauelement daher um einen Faktor von einigen 10.000 größer sein als in einem makroskopischen Bauelement. Realistisch hingegen ist eher ein Faktor 10 bis 100. Somit erhält man induzierte Spannungen (maximum open circuit voltage) in der Größenordnung von wenigen Millivolt. Hinsichtlich Leistung gewinnt man im Drahtwiderstand bei einem MEMS-Bauelement aufgrund der geringen Wicklungszahl etwa nochmal einen Faktor 10. Durch Kaskadierung mehrerer Spulen mit nur einer Wicklung kann man noch mal etwa einen weiteren Faktor 2 bis 3 gewinnen. Wenn man die Impedanz aufgrund der geringen Frequenz vernachlässigt, kann man die generierte Leistung grob über P = 1/8 U_{ind}² R mit mit R = Rₗₐₛₜ = R_{draht} abschätzen. Somit ergibt sich für das MEMS-Bauteil in den Figuren 6a bis 6c eine Leistung maximal etwa 1 mW.

Die Figuren 7a, 7b und 7c zeigen weitere Ausführungsbeispiele. Figur 7a zeigt beispielsweise einen Schritt des erfindungsgemäßen Verfahrens, bei dem in der der porösen Struktur 13 gegenüberliegenden Seite des Substrats 10, d.h. in dem Boden 11a der in dem Substrat 10 vorgesehenen Aussparung 11, eine Öffnung 71 in das Substrat 10 eingebracht wird, wobei die Öffnung 71 eine Fluidverbindung zwischen der Aussparung 11 und der Umgebung 15 bereitstellt.

In dem Hohlraum 16, der zwischen der porösen Struktur 13 und den Innenseiten 11a, 11b, 11c der in dem Substrat 10 vorgesehenen Aussparung 11 gebildet wird, befindet sich ein Formkörper 21. Der Formkörper 21 weist hier eine im Wesentlichen rechteckige Form auf.

Wie in Figur 7a zu erkennen ist, ist der Querschnitt der Öffnung 71 kleiner als der Formkörper 21, sodass die Öffnung 71 mittels des Formkörpers 21 verschließbar ist, um die Fluidverbindung zur Umgebung 15 zu unterbinden.

Figur 7b zeigt eine ähnliche Ausführungsform, wobei zwei weitere Öffnungen 72, 73 in das Substrat 10 eingebracht werden, wobei alle drei Öffnungen 71, 72, 73 eine Fluidverbindung zwischen der Aussparung 11 und der Umgebung 15 bereitstellen. In dem in Figur 7b abgebildeten Ausführungsbeispiel ist außerdem, wie zuvor beschrieben, eine Passivierungsschicht 17 auf der porösen Struktur 13 aufgebracht, wobei sich die Passivierungsschicht 17 vollständig über die poröse Struktur 13 erstreckt.

In Figur 7a hingegen ist zwar auch eine Passivierungsschicht 17 auf der porösen Struktur 13 aufgebracht. Die Passivierungsschicht 17 erstreckt sich jedoch nur abschnittsweise über die poröse Struktur 13. Ein Bereich bzw. ein Abschnitt 74 in der Mitte der porösen Struktur 13 ist von der Passivierungsschicht 17 ausgespart.

Bei den bisherigen Ausführungsbeispielen wurden also die im Hohlraum 16 eingeschlossenen beweglichen Partikel 12b bzw. Formkörper 21, 22 durch eine Passivierungsschicht 17 von der Umgebung 15 isoliert (Figur 1d). Wie in Figur 7b gezeigt, kann es jedoch auch von Vorteil sein, dem Umgebungsmedium ein Durchströmen des Hohlraums 16 zu ermöglichen. Dafür kann auf das Versiegeln des Hohlraums 16 verzichtet werden, oder die poröse Struktur 13 kann nur abschnittsweise mit der Passivierungsschicht 17 überdeckt werden.

Alternativ können auch neue Zugangsöffnungen 74 erzeugt werden, nachdem die Struktur gemäß den Figuren 1a bis 1e fertiggestellt wurde, z.B. mittels Ätzen des Substrats 10 und/oder der Versiegelungs- bzw. Passivierungsschicht 17.

Eine Anwendung wären beispielsweise Mikroventile. In den Figuren 7a und 7b sind zwei mögliche Ausführungsbeispiele schematisch dargestellt. Für die einfache Ausführung in Figur 7a können zusätzlich zu dem in den Figuren 1 bis 1e beschriebenen Herstellungsablauf eine Spule 61 und zur Ankopplung des Mediums der Umgebung 15 zwei Öffnungen 71, 74 im Substrat 10 bzw. in der Versiegelungsschicht 17 erzeugt werden. Der Formkörper 21 verschließt die untere, im Substrat 10 ausgebildete, Öffnung 71 und stellt somit eine Ventilfunktionalität bereit.

Im Ruhezustand, d.h. ohne Energiezufuhr, ist das Ventil 21, 71 geschlossen. Das durch die obere, in der Passivierungsschicht 17 ausgebildete, Eintrittsöffnung 74 (gekennzeichnet mit "in") eintretendes Fluid drückt den magnetischen Formkörper 21 gegen die untere Austrittsöffnung 71 (gekennzeichnet mit "out"). Fließt ein elektrischer Strom durch die Spule 61 wird der Formkörper 21 nach oben, d.h. in Richtung der Passivierungsschicht 17, gezogen und gibt die untere Austrittsöffnung 71 frei. Die Spule 61 kann permanent betätigt sein, um das Ventil 21, 71 offenzuhalten.

In dem Ausführungsbeispiel gemäß Figur 7b weist die poröse Struktur 13 ein magnetisches Material auf. Hier besteht die verfestigte poröse Struktur 13 aus weich- oder hartmagnetischen Partikeln 12. Prinzipiell kann die Vielzahl loser Partikel 12, die vor dem Entfernen selbiger in dem Hohlraum 16 angeordnet ist, Partikel aufweisen, die ein magnetisches Material aufweisen. Die mittels des Beschichtungsprozesses entstehende poröse Struktur 13 weist dann ebendiese magnetischen Partikel auf, so dass die poröse Struktur 13 selbst magnetische Eigenschaften aufweist.

Im Falle hartmagnetischer Partikel können diese zusammen mit dem magnetischen Formkörper 21 aufmagnetisiert werden. Wie in dem Ausführungsbeispiel gemäß Figur 7a ist das aus dem Formkörper 21 und der mittels des Formkörpers 21 verschließbaren Öffnung 71 gebildete Ventil 21, 71 im Ruhezustand geschlossen. Durch die Eintrittsöffnungen 72, 73 (gekennzeichnet mit "in") eintretendes Fluid drückt den magnetischen Formkörper 21 gegen die Austrittsöffnung 71 (gekennzeichnet mit "out").

Fließt ein elektrischer Strom durch die Spule 61 wird der Formkörper 21 nach oben, d.h. in Richtung der porösen Struktur 13, gezogen und gibt die Austrittsöffnung 71 somit frei. Aufgrund der magnetischen Anziehung zwischen der magnetische Partikel beinhaltenden porösen Struktur 13 und dem Formkörper 21 verbleibt letzterer auch nach Abschalten der Spule 61 an der Unterseite der porösen Struktur 13.

Zum Schließen des Ventils 21, 71 wird Spule 61 mit umgekehrter Polarität angesteuert, um abstoßende elektromagnetische Kräfte zu erzeugen. Sind diese ausreichend groß, löst sich der Formkörper 21 von der porösen Struktur 13 und verschließt die Austrittsöffnung 71 wieder. Aufgrund der sich aufbauenden Druckdifferenz zwischen "in" und "out" verbleibt der Formkörper 21 auch ohne weitere Energiezufuhr in dieser Position.

In den in den Figuren 7a und 7b gezeigten Ausführungsbeispielen kann anstelle einer integrierten Spule ebenso eine externe hybrid montierte Spule verwendet werden. Des Weiteren können Form und Größe der Aussparung 11 und des magnetischen Formkörpers 21, die Anordnung der Spulen 61 sowie Lage und Anzahl der Ein- bzw. Austrittsöffnungen 71, 72, 73, 74 variieren.

Figur 7c zeigt ein weiteres Ausführungsbeispiel. Hier weist das Substrat 10 ebenfalls eine Öffnung 71 auf, die auf der der porösen Struktur 13 gegenüberl iegenden Seite des Substrats 10 vorgesehen ist. Im Vergleich zu den Figuren 7a und 7b ist diese Öffnung 71 jedoch eine Eintrittsöffnung und daher mit "in" gekennzeichnet.

Die Passivierungsschicht 17 spart wiederum einen Abschnitt der porösen Struktur 13 aus. Dadurch wird eine Öffnung 74 gebildet, die im Vergleich zu den Figuren 7a und 7b nun jedoch eine Austrittsöffnung ist und daher mit "out" gekennzeichnet ist.

In dem Hohlraum 16 befindet sich ein kugelförmiger Formkörper 21 der größer ist als der Querschnitt der in dem Substrat 10 vorgesehenen Öffnung 71, so dass der Formkörper 21 die Öffnung 71 verschließen kann. Dies stellt wiederum eine Ventilfunktionalität bereit.

Mit der in Figur 7c gezeigten Ausführungsform wäre zum Beispiel eine weitere Anwendung in Form eines Durchfluss-Sensors bzw. -Reglers denkbar. Auch hier werden zusätzlich zu dem in den Figuren 1a bis 1d dargestellten Prozess mindestens eine Spule 61 erzeugt sowie Öffnungen 71 im Substrat 10 bzw. in der Versiegelungsschicht 17 hergestellt.

Fließt ein Fluid von unten nach oben (d.h. von dem Boden 11a der Aussparung 11 hin zu der porösen Struktur 13) durch die Aussparung 11 bzw. den Hohlraum 16 hindurch, wird der magnetische Formkörper 21 nach oben (d.h. in Richtung der porösen Struktur 13) gedrückt bzw. zum Schweben gebracht.

Die Lageänderung des magnetischen Formkörpers 21 führt zu einer Veränderung der Induktivität bzw. Resonanzfrequenz der Spule 61, die mittels eines schwachen, höherfrequenten Wechselspannungssignals leicht gemessen werden kann. Unabhängig davon kann mittels eines Gleichstroms durch dieselbe Spule 61 die Lage des magnetischen Formkörpers 21 beeinflusst werden, wodurch auch eine Durchflussregelung möglich wird.

Wie unter Bezugnahme auf die Figuren 7a bis 7c diskutiert, kann es also von Vorteil sein, den Hohlraum 16 nicht komplett mittels der Passivierungsschicht 17 zu versiegeln, oder eben nach dem Versiegeln neue Öffnungen 71, 72, 73, 74 im Substrat 10 oder der versiegelten porösen Struktur 13 zu erzeugen, um das Ein- bzw. Durchströmen eines flüssigen oder gasförmigen Mediums in bzw. durch den Hohlraum 16 und eine Interaktion mit den darin eingeschlossenen Partikeln 12 oder Formkörpern 21, 22 zu ermöglichen

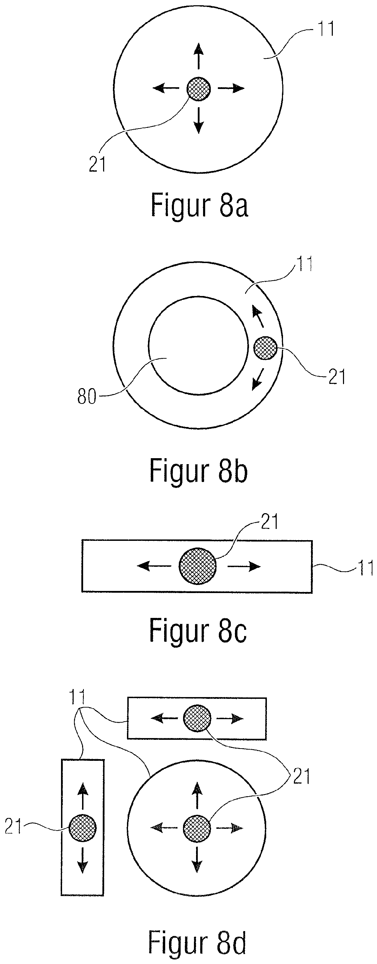

Die Figuren 8a bis 8d zeigen mögliche Ausgestaltungen von in das Substrat 10 einzubringenden Aussparungen 11. In Figur 8a ist eine Aussparung 11 gezeigt, die eine vollständige kreisförmige Ausgestaltung aufweist. In der kreisförmigen Aussparung 11 ist ein Formkörper 21 angeordnet, der sich in alle Richtungen (siehe Bewegungspfeile) bewegen kann.

Figur 8b zeigt eine weitere kreisförmige Ausgestaltung der Aussparung 11, wobei diese nicht unterbrechungsfrei ist. Diese Aussparung ist in Form eines Toroids bzw. eines Donuts ausgebildet, wobei lediglich der äußere Umfang der Aussparung 11 eine Vertiefung bildet, in der sich der Formkörper 21 bewegen kann (siehe Bewegungspfeile). In der Mitte 8 befindet sich keine Aussparung. Dieser Teil wird in der Regel aus dem Substratmaterial gebildet werden.

Figur 8c zeigt eine langgestreckte Aussparung 11. Hier weist die Aussparung 11 die Form eines Rechtecks auf. Der darin befindliche Formkörper 21 kann sich entlang des Rechtecks (siehe Bewegungspfeile) bewegen.

Figur 8d zeigt eine Kombination der soeben beschriebenen Formen von Aussparungen 11 mit darin angeordneten Formkörpern 21.

Wie bereits zuvor erwähnt, können die magnetischen Partikel 12 bzw. Formkörper 21, 22 im demagnetisierten Zustand verarbeitet und erst gegen Ende des Herstellungsprozesses an einer dafür geeigneten Stelle, beispielsweise vor dem Herausätzen der Partikel 12a aus Material A, aufmagnetisiert werden.

Das Magnetisieren kann aber auch nach dem Herausätzen der Partikel 12a aus dem Material A geschehen. Wird z.B. nach dem Herausätzen noch eine Passivierungsschicht z.B. mittels PECVD bei 300°C aufgebracht, würde die vorher ausgeführte Magnetisierung wieder verloren gehen und müsste wiederholt werden

Die gemäß Figur 1a zu Anfang des Herstellungsprozesses geätzte Aussparung 11 kann je nach Anwendung die unterschiedlichsten Formen aufweisen. Die Figuren 8a bis 8d zeigen einige grundlegende Möglichkeiten. Für eine gleichmäßige Empfindlichkeit in der Ebene ist eine Disc- oder Donut-Form (Figuren 8a bzw. 8b) besonders geeignet. Aber auch längliche Anordnungen können interessant sein, wenn Bewegungen oder Beschleunigungen in bestimmten Richtungen detektiert werden sollen (Figur 8c). Des Weiteren können mehrere Sensorelemente mit Aussparungen 11 unterschiedlicher Geometrie in einem Sensorarray kombiniert werden (Figur 8d).

Ein mögliches Anwendungsgebiet für die zuvor in sämtlichen Figuren beschriebenen Ausführungsbeispiele sind z.B. Bewegungssensoren aller Art. Im einfachsten Fall sind das z.B. Wake-Up-Sensoren zur Aktivierung elektronischer Schaltungen und Geräte oder Sensoren zur Detektion von Körperbewegungen wie z. B. Schrittzähler. Ein weiteres Anwendungsgebiet ist die Erfassung niederfrequenter und/oder nichtperiodischer Schwingungen oder Erschütterungen, die z.B. Verschleißeffekte an Maschinenteilen signalisieren. Aber auch Beschleunigungen könnten detektiert werden, wenn eine ausreichend hohe Auflösung der Positionen bzw. der Trajektorien der magnetischen Partikel 12b oder Formkörper 21, 22 innerhalb des Hohlraums 16 gewährleistet werden kann.

Weitere denkbare Realisierungen wären zum Beispiel elektromagnetische Energy-Harvester, die mechanische Energie ausnutzen. Diese beruhen im einfachsten Fall auf einer federnd gelagerten Masse, deren mechanische Oszillation, angeregt durch eine externe, periodische Schwingung, z.B. unter Verwendung des elektromagnetischen oder piezoelektrischen Prinzips in elektrische Energie umgewandelt wird. Solche Systeme funktionieren jedoch nur dann effektiv, wenn die Anregungsschwingung nahe der Resonanzfrequenz der beweglichen Masse liegt. Konventionell hergestellte elektromagnetische Vibrations-Harvester dieser Art werden z.B. für Frequenzen von 25 bis 120 Hz angeboten [3].

Bei mikromechanischen resonanten Harvestern liegt der Arbeitsbereich zumeist oberhalb von 50 Hz. Es ist technologisch schwierig, geringere Resonanzfrequenzen aufgrund der begrenzten Masse einer MEMS-Struktur zu realisieren. Erschwerend kommt hinzu, dass es sich im niederfrequenten Bereich zumeist um nichtperiodische Schwingungen handelt. Infolgedessen bleibt ein weiter Bereich der verfügbaren Schwingungsquellen (human motion, civil infrastructure, buildings, cars usw.) ungenutzt [4].

Eine bessere Effektivität im niederfrequenten Bereich wird erreicht, wenn die bewegliche Masse, einmal ausgelenkt, resonant nachschwingen kann. Ein Beispiel dafür ist der in [5] beschriebene elektromagnetische PFIG-Harvester. Er beinhaltet einen Magneten, der durch ausreichend starke Stöße zwischen zwei stabilen Positionen hin und her bewegt werden kann. In jeder dieser Positionen wird durch den mechanischen Impuls beim Umschalten ein weiterer Magnet in Schwingungen versetzt, wodurch in einer Spule ein Strom induziert wird. Weitere bistabile Systeme auf der Basis von Permanentmagneten sind z. B. in [6] beschrieben.

Eine Alternative zu den resonanten Systemen bilden Harvester, deren Masse frei beweglich ist, d.h. eine Aufhängung vollständig fehlt. Der Hauptvorteil besteht darin, dass diese nicht nur in einem schmalen Band um die Resonanzfrequenz sondern über einen weiten Frequenzbereich gleichermaßen effektiv funktionieren. Ein Beispiel ist der in [7] beschriebene elektromagnetische Harvester zur Energieversorgung von Autoschlüsseln, bestehend aus einem Permanentmagneten, der sich zwischen zwei herkömmlichen Leiterplatten mit Spulen frei bewegen kann. Die induzierte Spannung betrug in Abhängigkeit von der Geometrie der Spulen und der Größe des Magneten maximal 1,1 V. Generell wurde eine erhebliche Abnahme der induzierten Spannung mit der Größe des Magneten beobachtet. Vergleichbare MEMS-Bauelemente wurden noch nicht vorgestellt.

Die in den Figuren 5a und 5b dargestellten Bewegungssensoren könnten als nichtresonante Harvester im Sinne der oben genannten Beispiele genutzt werden, wenn anstelle des Detektors oder Detektorarrays 51 eine Spule 61 oder ein Spulenarray 62 tritt (siehe Figuren 6a und 6b). Die zwei Grundausführungen mit einseitig integrierter, planarer bzw. hybrid montierter, dreidimensionaler Spule sind in den Figuren 6a und 6b dargestellt und wurden bereits beschrieben. Es wäre jedoch wenig sinnvoll, die Anordnung aus [7] so wie sie ist auf ein MEMS-Bauelement zu übertragen. Schon aufgrund der begrenzten Anzahl der Windungen einer Planarspule würde die induzierte Spannung viel geringer ausfallen als in [7].

Zusammenfassend sei noch einmal erwähnt, dass ein Aspekt der vorliegenden Erfindung darauf aufbaut, dass die unterhalb der porösen Struktur 13 befindlichen unbeschichtet gebliebenen Partikel 12b oder Formkörper 21, 22 gemäß Figur 1d durch die poröse Struktur 13 hindurch selektiv herausgeätzt werden können. Dadurch entsteht ein Hohlraum 16 im Substrat 10, der auf der Oberseite durch die verfestigte poröse Struktur 13 und auf den anderen Seiten durch das Substratmaterial begrenzt wird. Wurde ein Gemisch aus Partikeln 12, beinhaltend Partikel 12a des Materials A mit Partikeln 12b des Materials B in die Aussparung 11 eingebracht, können die unbeschichtet gebliebenen Partikel 12a des Materials A selektiv zu den unbeschichtet gebliebenen Partikeln 12b des Materials B herausgeätzt werden. Letztere verbleiben im Hohlraum 16 unterhalb der porösen Struktur 13 und können sich dort frei bewegen. Die erfindungsgemäße Vorgehensweise ist in den Figuren 1a bis 1d schematisch dargestellt. In eine in dem Substrat 10 vorgesehene Kavität bzw. Aussparung 11 gemäß Figur 1a wird eine Vielzahl von Partikeln 12, d.h. ein Gemisch aus Partikeln 12a, 12b der Materialien A und B eingebracht (Figur 1b). Nun werden die Partikel 12 im oberen Bereich durch eine ALD-Abscheidung verfestigt (Figur 1c), wobei Hohlräume zwischen den verfestigten Partikeln 12 weitestgehend erhalten bleiben.

Die Partikel 12a des Materials A werden nun selektiv zu den Partikeln 12b des Materials B durch die poröse Struktur 13 hindurch herausgeätzt. Letztere verbleiben im Hohlraum 16 unter der porösen Struktur 13, wo sie sich bewegen können, je nachdem, wieviel Platz durch das Herausätzen der Partikel 12a vom Typ A entstanden ist (Figur 1d). Durch Aufbringen einer Schicht 17 auf der porösen Membran 13 kann der Hohlraum 16 mit den Partikeln 12b des Materials B verschlossen werden (Figur 1c), bei Bedarf hermetisch unter Vakuum oder in einer bestimmten Atmosphäre.

Das Material B kann nicht nur von Partikeln 12b sondern generell auch durch einzelne oder mehrere dreidimensionale Körper 21, 22 unterschiedlichster Anzahl, Form und Größe repräsentiert werden, sofern diese in die (z.B. geätzte) Kavität bzw. Aussparung 11 hineinpassen (siehe Figuren 2a und 2b). Die Formkörper 21, 22 oder Partikel 12b aus Material B können vor dem Befüllen der Kavität 11 mit den Partikeln 12a aus Material A vermischt werden. Sie können jedoch auch nacheinander in die Kavität 11 eingebracht werden. z. B. wenn der Formkörper 12b aus Material B sehr groß ist. Das Verfahren kann auf mehr als zwei Materialien erweitert werden. d. h. im Falle mehrerer Formkörper 21, 22 können diese aus unterschiedlichen Materialien B, C, D usw. bestehen bzw. die eingebrachten Partikel 12 können ein Gemisch aus den Materialien A bis C bestehen.

Die Vorgehensweise gemäß den Figuren 1 bis 1e erlaubt es, dreidimensionale Körper 21, 22 unterschiedlichster Anzahl, Form und Größe, sowohl einzelne größere Gebilde als auch eine Vielzahl kleiner Partikel 12 in definierten Hohlräumen 16 in einem Substrat 10 einzuschließen.

Die dreidimensionalen Körper 21, 22 sind innerhalb der Hohlräume 16 frei beweglich. Lage oder Anordnung, Größe oder Form, chemisch-physikalische Eigenschaften oder Zusammensetzung der dreidimensionalen Körper 21, 22 können sich verändern unter dem Einfluss z.B.:
a) äußerer elektromagnetischer Felder bzw. Strahlungen aller Art, oder
b) von Temperatur, oder
c) von Bewegungen bzw. Beschleunigungen oder der Gravitation, oder
d) eines Mediums, das in den abgeschlossenen Hohlraum 16 eindringt bzw. diesen durchströmt, z.B. durch die poröse Struktur 13 hindurch und/oder durch separat erzeugte Öffnungen 71, 72, 73, 74.

Das kann für verschiedene Sensor- oder Aktuatoranordnungen ausgenutzt werden.
Eine Vorrichtung gemäß vorangehend beschriebener Ausführungsbeispiele kann ferner eine Elektrodenstruktur aufweisen, die mit dem ersten Anteil und/oder dem zweiten Anteil von Partikeln kontaktiert ist und ausgebildet ist, um ein elektrisches Potenzial zwischen Elektroden der Elektrodenstruktur anzulegen oder zu erfassen, das bedeutet, ein elektrisches Potenzial an oder zwischen den Partikeln kann erzeugt oder erfasst werden.

Vorangehend beschriebene Ausführungsbeispiele sind von einer Größe (beispielsweise Durchmesser oder laterale Abmessung) unabhängig. Sind die Partikel 12 beispielsweise als Aluminiumoxidpartikel (Splitter) ausgeführt, können diese einen Durchmesser von beispielsweise zwischen 1 und 20 µm, 2 und 15 µm oder zwischen 5 und 12 µm aufweisen. Werden beispielsweise Silizium-Partikel verwendet, können diese beispielsweise einen Durchmesser oder eine laterale Abmessung in einem Bereich von 0,1 µm bis 20 µm, 0,5 µm bis 5 µm oder 0,8 µm bis 1,2 µm aufweisen.

Obwohl vorangehend beschriebene Ausführungsbeispiele so beschrieben wurden, dass die poröse Struktur 13 Hohlräume aufweist, die zumindest teilweise miteinander verbunden sind, so dass die poröse Struktur 13 eine poröse Membran bildet, kann, insbesondere bei einer Partikelgröße von <2 µm, mittels des Beschichtungsprozesses auch erreicht werden, dass die poröse Struktur 13 abgedichtet ist, d.h. die Hohlräume zwischen den Partikeln 12 verschlossen sind. So kann mittels der Beschichtung eine Aussparung 11 auch verschlossen werden.

Obwohl vorangehend beschriebene Ausführungsbeispiele so beschrieben wurden, dass das Substrat 10 eine ebene Form aufweist, kann das Substrat 10 auch andere Formen aufweisen. Bspw. kann das Substrat 10 eine gekrümmte Form (etwa eine Kuppelstruktur) oder eine abschnittsweise planare und/oder geknickte Form aufweisen.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Die beschriebenen Verfahrensschritte können auch in einer beliebigen anderen als der in den Patentansprüchen aufgeführten Reihenfolge ausgeführt werden.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

## Patentansprüche

1. Verfahren zum Herstellen einer Vorrichtung mit folgenden Schritten:
Bereitstellen eines Substrats (10), das eine Aussparung (11) aufweist;
Einbringen einer Vielzahl loser Partikel (12) in die Aussparung (11);
Beschichten eines ersten Anteils (1) der Partikel (12), so dass der erste Anteil (1) zu einer verfestigten porösen Struktur (13) verbunden wird, unter Verwendung eines Beschichtungsprozesses, der eine Eindringtiefe von einer Öffnung (11d) der Aussparung (11) ausgehend entlang einer Tiefenrichtung (14) in die Aussparung (11) hinein aufweist;
wobei die Eindringtiefe des Beschichtungsprozesses in die Aussparung (11) so eingestellt wird, dass ein zweiter Anteil (2) der Partikel (12) nicht mittels der Beschichtung verbunden wird, so dass der verfestigte erste Anteil (1) der Partikel (12) zwischen dem zweiten Anteil (2) der Partikel (12) und einer Umgebung (15) angeordnet ist; und
selektives zumindest teilweises Entfernen des in der Aussparung (11) eingeschlossenen unbeschichtet gebliebenen zweiten Anteils (2) der Partikel (12) aus der Aussparung (11) unter Beibehaltung der porösen Struktur (13), wobei sich zwischen der in dem Substrat (10) vorgesehenen Aussparung (11) und dem zu der porösen Struktur (13) verfestigten ersten Anteil (1) der Partikel (12) ein Hohlraum (16) ausbildet.

2. Verfahren nach Anspruch 1, wobei mindestens 10%, bevorzugt mindestens 25% und mehr bevorzugt mehr als 50% des zweiten Anteils (2) der Partikel (12) aus der Aussparung (11) entfernt werden, oder wobei der zweite Anteil (2) der Partikel (12) vollständig aus der Aussparung (11) entfernt wird.

3. Verfahren nach einem der vorhergehenden Ansprüche, wobei der zweite Anteil (2) der Partikel (12) selektiv mittels eines Ätzverfahrens entfernt wird, unter Verwendung eines Ätzmittels, das geeignet ist, um den zweiten Anteil (2) der Partikel (12) unter Beibehaltung der porösen Struktur (13) selektiv zu entfernen.

4. Verfahren nach einem der vorhergehenden Ansprüche, wobei der zweite Anteil (2) der Partikel (12) eine erste Art (12a) und eine zweite Art (12b) von Partikeln (12) aufweist, und wobei mittels eines geeigneten Ätzmittels selektiv eine bestimmte Art (12a, 12b) von Partikeln (12) entfernt wird.

5. Verfahren nach einem der vorhergehenden Ansprüche, wobei zuerst lose Partikel (12a), die ein erstes Material (A) aufweisen, und anschließend lose Partikel, die ein zweites Material (C) aufweisen, in die Aussparung (11) eingebracht werden, und wobei die das zweite Material (C) aufweisenden Partikel anschließend beschichtet werden, wobei die Beschichtung bezüglich der Tiefe so ausgeführt wird, dass sich der Prozess überwiegend auf die das zweite Material (C) aufweisenden Partikel beschränkt.

6. Verfahren nach einem der vorhergehenden Ansprüche, wobei eine Beschichtung (17), insbesondere eine Passivierungsschicht, auf die poröse Struktur (13) aufgebracht wird, sodass sich die Beschichtung (17) zumindest abschnittsweise über das Substrat (10) und/oder zumindest abschnittsweise über die poröse Struktur (13) erstreckt, oder wobei die Beschichtung (17) derart auf die poröse Struktur (13) aufgebracht wird, dass sie die poröse Struktur (13) vollständig bedeckt und die poröse Struktur (13) fluiddicht versiegelt.

7. Verfahren nach einem der vorhergehenden Ansprüche, wobei auf mindestens einer Innenseite (11a, 11b, 11c) der Aussparung (11) zumindest abschnittsweise eine Schicht aufgebracht wird, die Adhäsionskräfte und/oder Reibungskräfte zwischen der Aussparung (11) und einem nach dem zumindest teilweisen Entfernen des zweiten Anteils (2) der Partikel (12) in der Aussparung (11) verbleibenden Objekts (12b, 21, 22) verringert, und/oder eine Schicht, die von einem nach dem zumindest teilweisen Entfernen des zweiten Anteils (2) der Partikel (12) in der Aussparung (11) verbleibenden Objekts (12b, 21, 22) ausgehende Strahlung verringert.

8. Verfahren nach einem der vorhergehenden Ansprüche, wobei die Vielzahl loser Partikel (12) Partikel (12a, 12b) aufweist, die ein magnetisches Material aufweisen.

9. Verfahren nach einem der vorhergehenden Ansprüche, wobei der Beschichtungsprozess, mittels dem der erste Anteil (1) der Partikel (12) beschichtet wird, ein ALD-Prozess (Atomic Layer Deposition = Atomlagenabscheidung) ist.

10. Verfahren nach einem der vorhergehenden Ansprüche, wobei vor dem Beschichten des ersten Anteils (1) der Partikel (12) mindestens ein als Formkörper ausgebildetes Objekt (21, 22) zusätzlich zu der Vielzahl von losen Partikeln (12) in die Aussparung (11) eingebracht wird, wobei das mindestens eine Objekt (21, 22) ein von der Vielzahl von losen Partikeln (12) unterschiedliches Material aufweist, wobei die Vielzahl von losen Partikeln (12) ein Material aufweisen, das mittels eines Ätzmittels aus der Aussparung (11) entfernbar ist, während das Material des mindestens eine Objekt (21, 22) nicht auf das Ätzmittel reagiert.

11. Verfahren nach Anspruch 10, wobei das mindestens eine Objekt (21, 22) eine magnetische Eigenschaft aufweist, wobei das mindestens eine Objekt (21, 22) in einem demagnetisierten Zustand in die Aussparung (11) eingebracht wird und vor oder nach dem zumindest teilweisen Entfernen des zweiten Anteils (2) der Partikel (12) magnetisiert wird, und wobei eine Magnetfeldsensorik (51) an dem Substrat (10) angeordnet wird, um mittels der Magnetfeldsensorik (51) das Magnetfeld des mindestens einen Objekts (12b, 21, 22) zu bestimmen.

12. Verfahren nach Anspruch 11, wobei eine mit dem mindestens einen Objekt (21, 22) wechselwirkende Spule (61, 62) an dem Substrat (10) angeordnet wird, um mittels einer Relativbewegung zwischen der Spule (61, 62) und dem Formkörper (21, 22) eine sich in der Spule (61, 62) einstellende Induktionsspannung zu erzeugen, oder um mittels eines durch die Spule (61, 62) fließenden elektrischen Stroms eine magnetische Induktionskraft auf das mindestens eine Objekt (21, 22) auszuüben.

13. Verfahren nach einem der Ansprüche 12 bis 17, wobei in der der porösen Struktur (13) gegenüberliegenden Seite (11a) des Substrats (10) eine Öffnung (71) in das Substrat (10) eingebracht wird, die eine Fluidverbindung zwischen der Aussparung (11) und der Umgebung (15) bereitstellt, und wobei der Querschnitt der Öffnung (71) kleiner ist als das nach dem zumindest teilweisen Entfernen des zweiten Anteils (2) der Partikel (12) in der Aussparung (11) verbleibende Objekt (21, 22), sodass die Öffnung (71) mittels des Objekts (21, 22) verschließbar ist, um die Fluidverbindung zur Umgebung (15) zu unterbinden.

14. Vorrichtung mit
einem Substrat (10), das eine Aussparung (11) aufweist, und
einer porösen Struktur (13), die derart in oder an der Aussparung (11) angeordnet ist, dass zwischen der in dem Substrat (10) vorgesehenen Aussparung (11) und der porösen Struktur (13) ein Hohlraum (16) bestehen bleibt,
wobei die poröse Struktur (13) eine Vielzahl von Partikeln (12a) aufweist, die mittels einer Beschichtung miteinander verbunden und zu der porösen Struktur (13) verfestigt sind, und
wobei der Hohlraum (16) zu weniger als 90% seines Volumens mit losen Partikeln (12a) gefüllt ist, oder wobei der Hohlraum (16) keine dieser losen Partikel (12a) aufweist.

15. Vorrichtung nach Anspruch 14, wobei der Hohlraum (16) zu weniger als 75%, bevorzugt zu weniger als 50%, mehr bevorzugt zu weniger als 25% und besonders bevorzugt zu weniger als 10% seines Volumens mit den losen Partikeln (12a) gefüllt ist, oder wobei der Hohlraum (16) keine dieser losen Partikel (12a) aufweist.

16. Vorrichtung nach Anspruch 14 oder 15, wobei der zweite Anteil (2) der Partikel (12) eine erste Art (12a) und eine zweite Art (12b) von Partikeln (12) aufweist, und wobei mittels eines geeigneten Ätzmittels selektiv eine bestimmte Art (12a, 12b) von Partikeln (12) entfernbar ist.

17. Vorrichtung nach einem der Ansprüche 14 bis 16, wobei in dem Hohlraum (16) mindestens ein als Formkörper ausgebildetes Objekt (21, 22) angeordnet ist, wobei der Formkörper (21, 22) ein Material aufweist, das sich von dem Material der in dem Hohlraum (16) befindlichen losen Partikel (12) unterscheidet.

18. Vorrichtung nach Anspruch 17, wobei das mindestens eine Objekt (21, 22) eine magnetische Eigenschaft aufweist, wobei eine Magnetfeldsensorik (51) an dem Substrat (10) angeordnet ist, die ausgebildet ist, um das Magnetfeld des mindestens einen Objekts (21, 22) zu bestimmen.

19. Vorrichtung nach Anspruch 18, wobei eine mit dem mindestens einen Objekt (21, 22) wechselwirkende Spule (61, 62) an dem Substrat (10) angeordnet ist, die ausgebildet ist, um mittels einer Relativbewegung zwischen der Spule (61, 62) und dem mindestens einen Objekt (21, 22) eine sich in der Spule (61, 62) einstellende Induktionsspannung zu erzeugen, oder um mittels eines durch die Spule (61, 62) fließenden elektrischen Stroms eine magnetische Induktionskraft auf das mindestens eine Objekt (21, 22) auszuüben.

20. Vorrichtung nach einem der Ansprüche 14 bis 19, wobei die poröse Struktur (13) eine Vielzahl von Partikeln (12) mit magnetischer Eigenschaft aufweist.

21. Vorrichtung nach einem der Ansprüche 14 bis 20, wobei eine Beschichtung (17), insbesondere eine Passivierungsschicht, auf der porösen Struktur (13) aufgebracht ist, sodass sich die Beschichtung (17) zumindest abschnittsweise über das Substrat (10) und/oder zumindest abschnittsweise über die poröse Struktur (13) erstreckt, oder wobei die Beschichtung (17) die poröse Struktur (13) vollständig bedeckt und die poröse Struktur (13) fluiddicht versiegelt.

22. Vorrichtung nach einem der Ansprüche 17 bis 21, wobei in der der porösen Struktur (13) gegenüberliegenden Seite (11a) des Substrats (10) eine Öffnung (71) in dem Substrat (10) ausgebildet ist, die eine Fluidverbindung zwischen der Aussparung (11) und der Umgebung (15) bereitstellt, und wobei der Querschnitt der Öffnung (71) kleiner ist als das in dem Hohlraum (16) befindliche Objekt (21, 22), sodass die Öffnung (71) mittels des Objekts (21, 22) verschließbar ist, um die Fluidverbindung zur Umgebung (15) zu unterbinden.

## Claims

1. Method of producing a device, comprising:
providing a substrate (10) comprising a recess (11);
introducing a multitude of loose particles (12) into the recess (11);
coating a first portion (1) of the particles (12), so that the first portion (1) is connected to form a solidified porous structure (13), while using a coating process comprising a depth of penetration which extends from an opening (11d) of the recess (11), along a direction of depth (14), and into the recess (11);
wherein the depth of penetration of the coating process into the recess (11) is set such that a second portion (2) of the particles (12) is not connected by means of the coating, so that the solidified first portion (1) of the particles (12) is arranged between the second portion (2) of the particles (12) and surroundings (15); and
selectively removing from the recess (11), at least partly, the second portion (2) of the particles (12) which is enclosed in the recess (11) and has remained uncoated, while maintaining the porous structure (13), a cavity (16) forming between the recess (11) provided within the substrate (10) and that portion (1) of the particles (12) which has solidified to form the porous structure (13).

2. Method as claimed in claim 1, wherein at least 10%, preferably at least 25%, and more preferably more than 50% of the second portion (2) of the particles (12) are removed from the recess (11), or wherein the second portion (2) of the particles (12) is completely removed from the recess (11).

3. Method as claimed in any one of the preceding claims, wherein the second portion (2) of the particles (12) is selectively removed by means of an etching process which uses an etchant suitable for selectively removing the second portion (2) of the particles (12) while maintaining the porous structure (13).

4. Method as claimed in any one of the preceding claims, wherein the second portion (2) of the particles (12) comprises a first type (12a) and a second type (12b) of particles (12), and wherein a specific type (12a, 12b) of particles (12) is selectively removed by means of a suitable etchant.

5. Method as claimed in any one of the preceding claims, wherein loose particles (12a) comprising a first material (A) and subsequently, loose particles comprising a second material (C) are first introduced into the recess (11), and wherein the particles comprising the second material (C) are subsequently coated, the coating being performed, with regard to the depth, such that the process is predominantly limited to the particles comprising the second material (C).

6. Method as claimed in any one of the preceding claims, wherein a coating (17), in particular a passivation layer, is applied onto the porous structure (13), so that the coating (17) extends at least in portions across the substrate (10) and/or extends at least in portions across the porous structure (13), or wherein the coating (17) is applied onto the porous structure (13) such that it covers the entire porous structure (13) and seals off the porous structure (13) in a fluid-tight manner.

7. Method as claimed in any one of the preceding claims, wherein at least one inner side (11a, 11b, 11c) of the recess (11) has applied onto it, at least in portions, a layer which reduces adhesive forces and/or frictional forces between the recess (11) and an object (12b, 21, 22) which remains within the recess (11) after the second portion (2) of the particles (12) has been at least partly removed, and/or a layer which reduces the radiation emanating from an object (12b, 21, 22) which remains within the recess (11) after the second portion (2) of the particles (12) has at least been partly removed.

8. Method as claimed in any one of the preceding claims, wherein the plurality of loose particles (12) comprise particles (12a, 12b) comprising magnetic materials.

9. Method as claimed in any one of the preceding claims, wherein the coating process by means of which the first portion (1) of the particles (12) is coated is an ALD (atomic layer deposition) process.

10. Method as claimed in any one of the preceding claims, wherein, prior to coating of the first portion (1) of the particles (12), at least one object (21, 22) formed as a molded body is introduced into the recess (11) in addition to the multitude of loose particles (12), wherein the at least object (21, 22) comprises a material different from that of the multitude of loose particles (12), wherein the multitude of loose particles (12) comprise a material which may be removed from the recess (11) by means of an etchant, whereas the material of the at least one object (21, 22) does not react to the etchant.

11. Method as claimed in claim 10, wherein the at least one object (21, 22) comprises a magnetic property, wherein the at least one object (21, 22) is introduced into the recess (11) in a demagnetized state and is magnetized prior to or following the at least partial removal of the second portion (2) of the particles (12), and wherein a magnetic field sensor system (51) is arranged on the substrate (10) so as to determine the magnetic field of the at least one object (12b, 21, 22) by means of the magnetic field sensor system (51).

12. Method as claimed in claim 11, wherein a coil (61, 62) interacting with the at least one object (21, 22) is arranged at the substrate (10) so as to generate, by means of a relative movement between the coil (61, 62) and the molded body (21, 22), an induced voltage which forms within the coil (61, 62), or to exert a magnetic induction force on the at least one object (21, 22) by means of an electric current flowing through the coil (61, 62).

13. Method as claimed in claim 12 to 17, wherein in that side (11a) of the substrate (10) which is located opposite the porous structure (13), the substrate (10) has an opening (71) introduced into it which provides a fluid connection between the recess (11) and the surroundings (15), and wherein the cross section of the opening (71) is smaller than the object (21, 22) which remains within the recess (11) once the second portion (2) of the particles (12) has been at least partly removed, so that the opening (71) may be closed by means of the object (21, 22) so as to suppress the fluid connection to the surroundings (15).

14. Device comprising
a substrate (10) comprising a recess (11), and
a porous structure (13) which is arranged within or at the recess (11) such that a cavity (16) remains between the recess (11) provided within the substrate (10) and the porous structure (13),
wherein the porous structure (13) comprises a multitude of particles (12a) which are connected to one another by means of a coating and are solidified to form the porous structure (13), and
wherein less than 90% of the volume of the cavity (16) is filled with loose particles (12a), or wherein the cavity (16) does not comprise any of these loose particles (12a).

15. Device as claimed in claim 14, wherein less than 75%, preferably less than 50%, more preferably less than 25%, and particularly preferably less than 10% of the volume of the cavity (16) is filled with the loose particles (12a), or wherein the cavity (16) comprises none of said loose particles (12a).

16. Device as claimed in claim 14 or 15, wherein the second portion (2) of the particles (12) comprises a first type (12a) and a second type (12b) of particles (12), and wherein a specific type (12a, 12b) of particles (12) is selectively removable by means of a suitable etchant.

17. Device as claimed in any one of claims 14 to 16, wherein the cavity (16) has at least one object (21, 22) arranged therein which is configured as a molded body, said molded body (21, 22) comprising a material different from the material of the loose particles (12) present within the cavity (16).

18. Device as claimed in claim 17, wherein the at least one object (21, 22) comprises a magnetic property, wherein a magnetic field sensor system (51) is arranged at the substrate (10) and is configured to determine the magnetic field of the at least one object (21, 22).

19. Device as claimed in claim 18, wherein a coil (61, 62) interacting with the at least one object (21, 22) is arranged at the substrate (10) and is configured to generate, by means of a relative movement between the coil (61, 62) and the at least one object (21, 22), an induced voltage which forms within the coil (61, 62), or to exert a magnetic induction force on the at least one object (21, 22) by means of an electric current flowing through the coil (61, 62).

20. Device as claimed in any one of claims 14 to 19, wherein the porous structure (13) comprises a multitude of particles (12) having a magnetic property.

21. Method as claimed in any one of claims 14 to 20, wherein a coating (17), in particular a passivation layer, is applied onto the porous structure (13), so that the coating (17) extends at least in portions across the substrate (10) and/or extends at least in portions across the porous structure (13), or wherein the coating (17) covers the entire porous structure (13) and seals off the porous structure (13) in a fluid-tight manner.

22. Device as claimed in any one of claims 17 to 21, wherein in that side (11a) of the substrate (10) which is located opposite the porous structure (13), the substrate (10) has an opening (71) formed therein which provides a fluid connection between the recess (11) and the surroundings (15), and wherein the cross section of the opening (71) is smaller than the object (21, 22) located within the cavity (16), so that the opening (71) may be closed by means of the object (21, 22) so as to suppress the fluid connection to the surroundings (15).

## Revendications

1. Procédé de fabrication d'un dispositif, aux étapes suivantes consistant à:
prévoir un substrat (10) qui présente un évidement (11);
introduire une pluralité de particules libres (12) dans l'évidement (11);
revêtir une première part (1) des particules (12) de sorte que la première part (1) soit assemblée pour former une structure poreuse solidifiée (13), à l'aide d'un procédé de revêtement qui présente une profondeur de pénétration en partant d'une ouverture (11d) de l'évidement (11) dans une direction de profondeur (14) dans l'évidement (11);
dans lequel la profondeur de pénétration du processus de revêtement dans l'évidement (11) est réglée de sorte qu'une deuxième part (2) des particules (12) ne soit pas assemblée au moyen du revêtement, de sorte que la première part solidifiée (1) des particules (12) soit disposée entre la deuxième part (2) des particules (12) et un environnement (15); et
éliminer de l'évidement (11) de manière sélective au moins partiellement la deuxième part (2) des particules (12) restée non revêtue enfermée dans l'évidement (11) tout en maintenant la structure poreuse (13), où entre l'évidement (11) prévu dans le substrat (10) et la première part (1) des particules (12) solidifiée pour former la structure poreuse (13) se forme une cavité (16).

2. Procédé selon la revendication 1, dans lequel au moins 10%, de préférence au moins 25% et plus préférablement plus de 50% de la deuxième part (2) des particules (12) sont éliminés de l'évidement (11), ou dans lequel la deuxième part (2) des particules (12) est entièrement éliminée de l'évidement (11).

3. Procédé selon l'une des revendications précédentes, dans lequel la deuxième part (2) des particules (12) est éliminée de manière sélective au moyen d'un procédé de gravure, à l'aide d'un agent de gravure qui convient pour éliminer de manière sélective la deuxième part (2) des particules (12) tout en maintenant la structure poreuse (13).

4. Procédé selon l'une des revendications précédentes, dans lequel la deuxième part (2) des particules (12) présente un premier type (12a) et un deuxième type (12b) de particules (12), et dans lequel est éliminé de manière sélective, au moyen d'un agent de gravure approprié, un type déterminé (12a, 12b) de particules (12).

5. Procédé selon l'une des revendications précédentes, dans lequel sont introduites dans l'évidement (11) tout d'abord des premières particules libres (12a) qui présentent un premier matériau (A) et ensuite des particules libres qui présentent un deuxième matériau (C) et dans lequel les particules présentant le deuxième matériau (C) sont ensuite revêtues, dans lequel le revêtement est réalisé en ce qui concerne la profondeur de sorte que le processus se limite principalement aux particules présentant le deuxième matériau (C).

6. Procédé selon l'une des revendications précédentes, dans lequel un revêtement (17), en particulier une couche de passivation, est appliqué sur la structure poreuse (13), de sorte que le revêtement (17) s'étende au moins par segments sur le substrat (10) et/ou au moins par segments sur la structure poreuse (13), ou dans lequel le revêtement (17) est appliqué sur la structure poreuse (13) de sorte qu'il recouvre entièrement la structure poreuse (13) et obture la structure poreuse (13) de manière étanche au fluide.

7. Procédé selon l'une des revendications précédentes, dans lequel est appliquée sur au moins une face intérieure (11a, 11b, 11c) de l'évidement (11) au moins par segments une couche qui réduit les forces d'adhérence et/ou les forces de friction entre l'évidement (11) et un objet (12b, 21, 22) restant dans l'évidement (11) après l'élimination au moins partielle de la deuxième part (2) des particules (12), et/ou une couche qui réduit le rayonnement partant d'un objet (12b, 21, 22) restant dans l'évidement (11) après l'élimination au moins partielle de la deuxième part (2) des particules (12).

8. Procédé selon l'une des revendications précédentes, dans lequel la pluralité de particules libres (12) présente des particules (12a, 12b) qui présentent un matériau magnétique.

9. Procédé selon l'une des revendications précédentes, dans lequel le processus de revêtement au moyen duquel est revêtue la première part (1) des particules (12) est un processus ALD (Atomic Layer Déposition = Dépôt de couche atomique).

10. Procédé selon l'une des revendications précédentes, dans lequel est introduit dans l'évidement (11), avant le revêtement de la première part (1) des particules (12), au moins un objet réalisé en forme de corps façonné (21, 22), en plus de la pluralité de particules libres (12), dans lequel l'au moins un objet (21, 22) présente un matériau différent de la pluralité de particules libres (12), dans lequel la pluralité de particules libres (12) présentent un matériau qui peut être éliminé de l'évidement (11) au moyen d'un agent de gravure, tandis que le matériau de l'au moins un objet (21, 22) ne réagit pas à l'agent de gravure.

11. Procédé selon la revendication 10, dans lequel l'au moins un objet (21, 22) présente une propriété magnétique, dans lequel l'au moins un objet (21, 22) est introduit dans l'évidement (11) dans un état démagnétisé et est magnétisé avant ou après l'élimination au moins partielle de la deuxième part (2) des particules (12) et dans lequel un système capteur de champ magnétique (51) est disposé sur le substrat (10) pour déterminer, au moyen du système capteur de champ magnétique (51), le champ magnétique de l'au moins un objet (12b, 21, 22).

12. Procédé selon la revendication 11, dans lequel une bobine interactive (61, 62) avec l'au moins un objet (21, 22) est disposée sur le substrat (10) pour générer, au moyen d'un mouvement relatif entre la bobine (61, 62) et le corps façonné (21, 22), une tension d'induction se produisant dans la bobine (61, 62), ou pour exercer, au moyen d'un courant électrique circulant dans la bobine (61, 62), une force d'induction magnétique sur l'au moins un objet (21, 22).

13. Procédé selon l'une des revendications 12 à 17, dans lequel est réalisée, dans le côté (11a) du substrat (10) opposé à la structure poreuse (13), une ouverture (71) dans le substrat (10) qui permet une communication de fluide entre l'évidement (11) et l'environnement (15), et dans lequel la section transversale de l'ouverture (71) est plus petite que l'objet (21, 22) restant dans l'évidement (11) après l'élimination au moins partielle de la deuxième part (2) des particules (12), de sorte que l'ouverture (71) puisse être fermée au moyen de l'objet (21, 22) pour empêcher la communication de fluide avec l'environnement (15).

14. Dispositif avec
un substrat (10) qui présente un évidement (11), et
une structure poreuse (13) qui est disposée dans ou sur l'évidement (11) de sorte que persiste une cavité (16) entre l'évidement (11) prévu dans le substrat (10) et la structure poreuse (13),
dans lequel la structure poreuse (13) présente une pluralité de particules (12a) qui sont assemblées entre elles par un revêtement et solidifiées pour obtenir la structure poreuse (13), et
dans lequel la cavité (16) est remplie de particules libres (12a) à moins de 90% de son volume ou, dans lequel la cavité (16) ne présente aucune de ces particules libres (12a).

15. Dispositif selon la revendication 14, dans lequel la cavité (16) est remplie des particules libres (12a) à moins de 75%, de préférence à moins de 50%, plus préférablement à moins de 25% et de manière particulièrement préférée à moins de 10% de son volume, ou dans laquelle la cavité (16) ne présente aucune de ces particules libres (12a).

16. Dispositif selon la revendication 14 ou 15, dans lequel la deuxième part (2) des particules (12) présente un premier type (12a) et un deuxième type (12b) de particules (12), et dans lequel peut être éliminé de manière sélective, au moyen d'un agent de gravure approprié, un type déterminé (12a, 12b) de particules (12).

17. Dispositif selon l'une des revendications 14 à 16, dans lequel est disposé dans la cavité (16) au moins un objet (21, 22) réalisé en forme de corps façonné, dans lequel le corps façonné (21, 22) présente un matériau qui diffère du matériau des particules libres (12) se trouvant dans la cavité (16).

18. Dispositif selon la revendication 17, dans lequel l'au moins un objet (21, 22) présente une propriété magnétique, dans lequel est disposé sur le substrat (10) un système capteur de champ magnétique (51) qui est conçu pour déterminer le champ magnétique de l'au moins un objet (21, 22).

19. Dispositif selon la revendication 18, dans lequel est disposée sur le substrat (10) une bobine interactive (61, 62) avec l'au moins un objet (21, 22) qui est conçue pour générer, au moyen d'un mouvement relatif entre la bobine (61, 62) et l'au moins un objet (21, 22), une tension d'induction se produisant dans la bobine (61, 62), ou pour exercer, au moyen d'un courant électrique circulant dans la bobine (61, 62), une force d'induction magnétique sur l'au moins un objet (21, 22).

20. Dispositif selon l'une des revendications 14 à 19, dans lequel la structure poreuse (13) présente une pluralité de particules (12) avec une propriété magnétique.

21. Dispositif selon l'une des revendications 14 à 20, dans lequel un revêtement (17), en particulier une couche de passivation, est appliqué sur la structure poreuse (13), de sorte que le revêtement (17) s'étende au moins par segments sur le substrat (10) et/ou au moins par segments sur la structure poreuse (13), ou dans lequel le revêtement (17) recouvre entièrement la structure poreuse (13) et obture la structure poreuse (13) de manière étanche aux fluides.

22. Dispositif selon l'une des revendications 17 à 21, dans lequel est formée dans le substrat (10), du côté (11a) du substrat (10) opposé à la structure poreuse (13), une ouverture (71) qui permet une communication de fluide entre l'évidement (11) et l'environnement (15), et dans laquelle la section transversale de l'ouverture (71) est plus petite que l'objet (21, 22) se trouvant dans la cavité (16), de sorte que l'ouverture (71) puisse être fermée au moyen de l'objet (21, 22) pour empêcher la communication de fluide avec l'environnement (15).
